(19) Europäisches Patentamt
European Patent Office
Office européen des brevets

(11) EP 2 668 503 B1

(12) **EUROPEAN PATENT SPECIFICATION**

(45) Date of publication and mention of the grant of the patent:
**24.06.2015 Bulletin 2015/26**

(21) Application number: **12702017.0**

(22) Date of filing: **30.01.2012**

(51) Int Cl.:
***G01N 33/574*** *(2006.01)*

(86) International application number:
**PCT/EP2012/051463**

(87) International publication number:
**WO 2012/101283 (02.08.2012 Gazette 2012/31)**

# (54) COMBINATORIAL BIOMARKERS FOR CLINICAL APPLICATIONS IN LUNG CANCER PATIENT MANAGEMENT

KOMBINATORISCHE BIOMARKER FÜR KLINISCHE ANWENDUNGEN IN EINEM LUNGENKREBS-PATIENTENMANAGEMENT

BIOMARQUEURS COMBINATOIRES DESTINÉS AUX APPLICATIONS CLINIQUES MISES EN UVRE POUR LA GESTION DES PATIENTS SOUFFRANTS DE CANCER DU POUMON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2011 US 201161437090 P**

(43) Date of publication of application:
**04.12.2013 Bulletin 2013/49**

(73) Proprietor: **F.Hoffmann-La Roche AG**
**4070 Basel (CH)**

(72) Inventors:
- **GUERGOVA-KURAS, Mariana**
  **F-92320 Chatillon (FR)**
- **KIEFFER, Yann**
  **F-94700 Maisons-alfort (FR)**
- **MALDERES-BLOES, Carole**
  **F-91240 Saint-Michel-sur-Orge (FR)**
- **KREMEURT, Alexandra**
  **F-91000 Evry (FR)**
- **TAKACS, Laszlo**
  **F-92340 Bourg la Reine (FR)**

(74) Representative: **Becker, Philippe et al**
**Cabinet Becker & Associés**
**25, rue Louis Le Grand**
**75002 Paris (FR)**

(56) References cited:
**WO-A1-2010/000467** **WO-A1-2010/130839**
**WO-A2-2008/092094** **US-A1- 2007 099 209**

- **Petra Stieber ET AL: "National Academy of Clinical Biochemistry Guidelines for the Use of Tumor Markers in Lung Cancer", NACB: Practice Guidelines And Recommendations For Use Of Tumor Markers In The Clinic, 1 January 2006 (2006-01-01), pages 1-29, XP055020923, Retrieved from the Internet: URL:http: //www.aacc.org/SiteCollectionDocu ments/ NACB/LMPG/tumor/chp3p_lung.pdf [retrieved on 2012-03-05]**
- **OKANO TETSUYA ET AL: "Plasma proteomics of lung cancer by a linkage of multi-dimensional liquid chromatography and two-dimensional difference gel electrophoresis", PROTEOMICS, WILEY - VCH VERLAG, WEINHEIM, DE, vol. 6, no. 13, 1 January 2006 (2006-01-01), pages 3938-3948, XP009140370, ISSN: 1615-9853**
- **HEO SUN-HEE ET AL: "Identification of putative serum glycoprotein biomarkers for human lung adenocarcinoma by multilectin affinity chromatography and LC-MS/MS", 20070101, vol. 7, no. 23, 1 January 2007 (2007-01-01), pages 4292-4302, XP009140371, DOI: 10.1002/PMIC. 200700433**
- **M. GUERGOVA-KURAS ET AL: "Discovery of Lung Cancer Biomarkers by Profiling the Plasma Proteome with Monoclonal Antibody Libraries", MOLECULAR & CELLULAR PROTEOMICS, vol. 10, no. 12, 26 September 2011 (2011-09-26), XP055026003, ISSN: 1535-9476, DOI: 10.1074/mcp.M111.010298**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Biosystems International

<120> Combinatorial biomarkers for clinical applications in lung cancer patient management

<130> B1123PC00

<160> 96

<170> PatentIn version 3.3

<210> 1
<211> 12
<212> PRT
<213> Peptide

<400> 1

```
Asn Asn Ser Tyr Leu Asp Glu Glu Gly Thr Trp Leu
1               5                   10
```

<210> 2
<211> 12
<212> PRT
<213> Peptide

<400> 2

```
Phe Pro Thr His Asn Pro Thr Ser Pro Leu Asp Met
1               5                   10
```

<210> 3
<211> 12
<212> PRT
<213> Peptide

<400> 3

```
Asp Leu Asn Asp Pro Pro Phe Phe Thr Ser Val Ser
1               5                   10
```

<210> 4
<211> 12
<212> PRT
<213> Peptide

<400> 4

```
Thr Thr His Trp Asp His Pro Phe Tyr Asp Asn Thr
1               5                   10
```

<210> 5
<211> 12
<212> PRT
<213> Peptide

<400> 5

```
Ala Trp His Pro Ser Ser Pro Leu Asp Tyr Gly Phe

      1               5                   10
```

<210> 6
<211> 12
<212> PRT
<213> Peptide

<400> 6

```
Ala Asn Ser Val Val Tyr Lys Pro Ser Ser Pro Leu
1               5                   10
```

<210> 7
<211> 12
<212> PRT
<213> Peptide

<400> 7

```
Thr Ser Tyr Asn Thr Asp His Pro Phe His Tyr Pro
1               5                   10
```

<210> 8
<211> 12
<212> PRT
<213> Peptide

<400> 8

```
Ser Pro Ser Ala Pro Ser Ser Pro Leu Asp Ser Met
1               5                   10
```

<210> 9
<211> 12
<212> PRT
<213> Peptide

<400> 9

Val Arg Ala Cys Val Asp Cys Asp Gln Pro Phe Tyr
1 5 10

<210> 10
<211> 12
<212> PRT
<213> Peptide

<400> 10

Ile Ala Pro Ser Ser Phe Leu Asp Glu Arg Thr Asp
1 5 10

<210> 11
<211> 12
<212> PRT
<213> Peptide

<400> 11

Thr Arg Pro His Thr Asp Pro Pro Phe Trp Trp Ala
1 5 10

<210> 12
<211> 12
<212> PRT
<213> Peptide

<400> 12

Glu Pro Asp Pro Pro Phe Tyr Thr His Leu Thr Ala
1 5 10

<210> 13
<211> 12
<212> PRT
<213> Peptide

<400> 13

Ser Gln Met Lys Pro Ser Ser His Leu Asp Trp Asp
1 5 10

<210> 14
<211> 12
<212> PRT

<213> Peptide

<400> 14

His Thr Leu Pro Gln Phe Leu Glu Trp His Lys Arg
1 5 10

<210> 15
<211> 12
<212> PRT
<213> Peptide

<400> 15

Gly Gln Val Val Phe Gln Asp Trp Leu Leu Val Arg
1 5 10

<210> 16
<211> 12
<212> PRT
<213> Peptide

<400> 16

His Glu Asn Ala Asn Lys Ile Ala Pro Trp Val Gln
1 5 10

<210> 17
<211> 12
<212> PRT
<213> Peptide

<400> 17

Gln Leu Lys Ile Ala Pro Trp Val Ile Glu Ser Pro
1 5 10

<210> 18
<211> 12
<212> PRT
<213> Peptide

<400> 18

Thr Asp Thr Leu Asp Ser Phe Leu Gly Arg Ser Pro
1 5 10

<210> 19
<211> 12
<212> PRT
<213> Peptide

<400> 19

Lys Pro Pro Arg Gln Val Val Ala Pro Trp Val Met
1 5 10

<210> 20
<211> 12
<212> PRT
<213> Peptide

<400> 20

Ile Ala Gln Thr Leu Pro Ala Phe Leu Ala Thr Arg
1 5 10

<210> 21
<211> 12
<212> PRT
<213> Peptide

<400> 21

Thr Glu Thr Leu His Glu Phe Leu Asp Gly Arg Lys
1 5 10

<210> 22
<211> 12
<212> PRT
<213> Peptide

<400> 22

Lys His Leu Glu His Pro Thr Tyr His Leu Trp His
1 5 10

<210> 23
<211> 12
<212> PRT
<213> Peptide

<400> 23

Ser Leu Asn Thr Ile Ala Pro Trp Ile Leu Thr Ala
1 5 10

<210> 24
<211> 12
<212> PRT
<213> Peptide

<400> 24

Lys Ala Thr Thr His Asn Ile Ala Trp Trp Val Ala
1 5 10

<210> 25
<211> 12
<212> PRT
<213> Peptide

<400> 25

His Asp Asn Ile Asn Met Ala Trp Trp Val Glu Trp
1 5 10

<210> 26
<211> 12
<212> PRT
<213> Peptide

<400> 26

Glu Trp Tyr Val Ser Thr Ser Leu Leu Pro Leu Pro
1 5 10

<210> 27
<211> 12
<212> PRT
<213> Peptide

<400> 27

His Ile Tyr Tyr Ile Ser Glu Ser Leu Leu Pro Met
1 5 10

<210> 28
<211> 12
<212> PRT
<213> Peptide

<400> 28

His Val Glu Tyr Glu Ile Ile Glu Gln Met Ile Tyr
1 5 10

<210> 29
<211> 12
<212> PRT
<213> Peptide

<400> 29

Ser Pro Phe Phe Tyr Thr Leu Thr Arg Val Pro Met
1 5 10

<210> 30
<211> 12
<212> PRT
<213> Peptide

<400> 30

Ser Ser Tyr Leu Tyr Thr Thr Gln Phe Val Pro Leu
1 5 10

<210> 31
<211> 12
<212> PRT
<213> Peptide

<400> 31

Thr Gly Trp Met Ile Ser Thr Thr Leu Ile Pro Thr
1 5 10

<210> 32
<211> 12
<212> PRT
<213> Peptide

<400> 32

Tyr Lys Tyr Ser Tyr Thr Thr Glu Leu Ile Leu Ile
1 5 10

<210> 33
<211> 12
<212> PRT
<213> Peptide

<400> 33

Ser Tyr Thr Tyr Thr Thr His Leu Leu Pro Leu Ala
1 5 10

<210> 34
<211> 12
<212> PRT
<213> Peptide

<400> 34

Thr Met Thr Gln Tyr Tyr Tyr Ser Thr Thr His Tyr
1 5 10

<210> 35
<211> 12
<212> PRT
<213> Peptide

<400> 35

Gln Phe Trp Thr Ile Thr Thr Thr Leu Val Pro His
1 5 10

<210> 36
<211> 12
<212> PRT
<213> Peptide

<400> 36

Gly Pro Lys Met Asp Ala Leu Ser Lys Ala Glu Asn
1 5 10

<210> 37
<211> 12
<212> PRT
<213> Peptide

<400> 37

Gly Pro Arg Trp Leu Asp Ala Leu Phe Met Ala Glu
1 5 10

<210> 38
<211> 12
<212> PRT
<213> Peptide

<400> 38

His Ile Gly Ala His Asp Ser Phe Glu Thr Lys His
1 5 10

<210> 39
<211> 12
<212> PRT
<213> Peptide

<400> 39

Asn Asn Ser Tyr Leu Asp Glu Glu Gly Thr Trp Leu
1 5 10

<210> 40
<211> 12
<212> PRT
<213> Peptide

<400> 40

Ala Met Asn Thr Val Val Cys Tyr Asp Glu Pro Cys
1 5 10

<210> 41
<211> 12
<212> PRT
<213> Peptide

<400> 41

Thr Asn Ser Pro Leu Asp Glu Leu Gly Thr His Pro
1 5 10

<210> 42
<211> 12

<212> PRT
<213> Peptide

<400> 42

Ser Thr Asn Ser His Glu His Trp Pro Met Ser Pro
1 5 10

<210> 43
<211> 12
<212> PRT
<213> Peptide

<400> 43

Asn His Phe Ser Ser Leu Gly Ile Leu Thr Ala Ala
1 5 10

<210> 44
<211> 12
<212> PRT
<213> Peptide

<400> 44

Ile Pro Gln Pro Ser Ile Leu Thr Thr Pro Met Leu
1 5 10

<210> 45
<211> 12
<212> PRT
<213> Peptide

<400> 45

Leu Arg Leu Pro Asn Thr Tyr Glu Asn Ala Lys Asn
1 5 10

<210> 46
<211> 12
<212> PRT
<213> Peptide

<400> 46

Gly Pro Leu Ser Pro Ala His Leu Ser Gln Arg Asn
1 5 10

<210> 47
<211> 12
<212> PRT
<213> Peptide

<400> 47

Phe Pro Leu Pro Phe Asp Pro Glu Met Leu Pro Leu
1 5 10

<210> 48
<211> 12
<212> PRT
<213> Peptide

<400> 48

Phe Pro Leu Pro Pro Glu Tyr Ser Met Lys Lys Asp
1 5 10

<210> 49
<211> 12
<212> PRT
<213> Peptide

<400> 49

Phe Lys Ile Pro Glu Gly Asn Asn Val Ser Val Leu
1 5 10

<210> 50
<211> 12
<212> PRT
<213> Peptide

<400> 50

Phe Arg Leu Pro Pro His Asp Ser Ser Asp His His
1 5 10

<210> 51
<211> 12
<212> PRT
<213> Peptide

<400> 51

Phe Phe Leu Pro His Ser Ala Thr Leu Pro Ser His
1 5 10

<210> 52
<211> 12
<212> PRT
<213> Peptide

<400> 52

Phe Ile Leu Pro His Arg Asn Gly Tyr Ile Asp Thr
1 5 10

<210> 53
<211> 12
<212> PRT

<213> Peptide

<400> 53

Leu Lys Leu Pro Asn Pro Asp Thr Ala Ala Pro Thr
1               5                   10

<210> 54
<211> 12
<212> PRT
<213> Peptide

<400> 54

Asn Asn Asn Met Gly Asn Ile Leu Leu Ile Thr Pro
1               5                   10

<210> 55
<211> 12
<212> PRT
<213> Peptide

<400> 55

Val Leu Leu Val Pro His His Ser His Gln Tyr Asp
1               5                   10

<210> 56
<211> 12
<212> PRT
<213> Peptide

<400> 56

Tyr Leu Lys Glu Ser Thr His Val Leu Leu Ala Ala
1               5                   10

<210> 57
<211> 12
<212> PRT
<213> Peptide

<400> 57

His Asp Asp Trp Tyr Ile Ser Thr Ser His Thr Asp
1               5                   10

<210> 58
<211> 12
<212> PRT
<213> Peptide

<400> 58

Ser Tyr Thr Tyr Thr Thr His Leu Leu Pro Leu Ala
1               5                   10

<210> 59

<211> 12
<212> PRT
<213> Peptide

<400> 59

Glu Trp Tyr Val Tyr Glu Lys Leu Phe Pro Leu Pro
1 5 10

<210> 60
<211> 12
<212> PRT
<213> Peptide

<400> 60

Tyr Lys Tyr Ser Tyr Thr Thr Glu Leu Ile Leu Ile
1 5 10

<210> 61
<211> 12
<212> PRT
<213> Peptide

<400> 61

His Gln Tyr Tyr Tyr Thr Ser Ser Leu Ile Tyr Thr
1 5 10

<210> 62
<211> 12
<212> PRT
<213> Peptide

<400> 62

Thr Ala Trp His Thr Thr Glu Glu Leu Ile Met Leu
1 5 10

<210> 63
<211> 12
<212> PRT
<213> Peptide

<400> 63

Asn Gly Tyr Ile Phe Thr Thr Gln Leu Ile Trp Ala
1 5 10

<210> 64
<211> 12
<212> PRT
<213> Peptide

<400> 64

Thr Val Asn Pro Leu Leu Arg Leu Leu Ser Met Gly
1 5 10

<210> 65
<211> 12
<212> PRT
<213> Peptide

<400> 65

Asn Asn Pro Met Leu Val Leu Leu Ser His Gly Ala
1 5 10

<210> 66
<211> 12
<212> PRT
<213> Peptide

<400> 66

Thr Val Pro Gln Pro Gln Arg Leu Thr Gln Lys Ser
1 5 10

<210> 67
<211> 12
<212> PRT
<213> Peptide

<900> 67

Ala Lys Tyr Glu Ser Leu Leu Arg Met Leu Ala Ala
1 5 10

<210> 68
<211> 12
<212> PRT
<213> Peptide

<400> 68

Gly His Glu Ser Gly Tyr His Ile Ser Glu Arg Ala
1 5 10

<210> 69
<211> 12
<212> PRT
<213> Peptide

<400> 69

Gly His Glu Glu His Leu Trp Gln Ile Leu His Phe
1 5 10

<210> 70

<211> 12
<212> PRT
<213> Peptide

<400> 70

Gly His Glu Glu Gln Arg Asn Trp Ser Met Glu Gly
1 5 10

<210> 71
<211> 12
<212> PRT
<213> Peptide

<400> 71

Ser Thr Tyr Thr Asp Met Leu Ala Asp Leu Ser Ala
1 5 10

<210> 72
<211> 12
<212> PRT
<213> Peptide

<400> 72

Ser Tyr Glu Ser Leu Leu Met Lys Leu Ala Arg Gly
1 5 10

<210> 73
<211> 12
<212> PRT
<213> Peptide

<400> 73

Tyr Arg Asp Ser Pro Gly Pro Arg Asp Lys Leu Leu
1 5 10

<210> 74
<211> 12
<212> PRT
<213> Peptide

<400> 74

Gly His Glu Glu Arg Glu Tyr Gln Met Met Leu Gln
1 5 10

<210> 75
<211> 12
<212> PRT

<213> Peptide

<400> 75

**Cys Leu Pro Tyr Pro Leu Cys Arg Gly Thr Ala Thr**
**1 5 10**

<210> 76
<211> 12
<212> PRT
<213> Peptide

<400> 76

**Thr Glu Cys Leu Pro Tyr Pro Leu Cys Leu Tyr Asn**
**1 5 10**

<210> 77
<211> 117
<212> PRT
<213> Peptide

<400> 77

**Glu Val Gln Leu Gln Gln Ser Gly Ala Asp Leu Val Lys Pro Gly Ala**
**1 5 10 15**

**Ser Val Lys Leu Ser Cys Thr Ala Ser Gly Phe Asn Ile Lys Asp Thr**
**20 25 30**

**Tyr Met His Trp Met Lys Gln Arg Pro Glu Gln Gly Leu Glu Trp Ile**
**35 40 45**

**Gly Arg Ile Asp Pro Ala Asn Gly Asn Ser Lys Tyr Asp Pro Lys Phe**
**50 55 60**

**Gln Gly Lys Ala Thr Ile Thr Ala Asp Thr Ser Ser Asn Thr Ala Tyr**
**65 70 75 80**

**Leu Gln Leu Ser Ser Leu Thr Ser Glu Asp Thr Ala Val Tyr Phe Cys**
**85 90 95**

**Thr Lys Ser Ala Gly Val Pro Phe Ala Tyr Trp Gly Gln Gly Thr Leu**
**100 105 110**

**Val Thr Val Ser Ala**
**115**

<210> 78
<211> 12
<212> PRT

<213> Peptide

<400> 78

Asn Glu Thr Trp Ser Glu Phe Trp Arg Leu His Asn
1 5 10

<210> 79
<211> 12
<212> PRT
<213> Peptide

<400> 79

Thr Ala Gln Asp Asp Arg Ile Phe His Met Met His
1 5 10

<210> 80
<211> 12
<212> PRT
<213> Peptide

<400> 80

Arg Gly Thr Gln Asp Met Glu Tyr Phe Ala Met His
1 5 10

<210> 81
<211> 118
<212> PRT
<213> Peptide

<400> 81

Glu Val Met Leu Val Glu Ser Gly Gly Gly Leu Val Lys Pro Gly Gly
1               5                   10                  15

Ser Leu Lys Leu Ser Cys Ala Ala Ser Glu Phe Thr Phe Ser Asn Tyr
            20                  25                  30

Ala Met Ser Trp Val Arg Gln Thr Pro Glu Lys Arg Leu Glu Trp Val
        35                  40                  45

Ala Thr Ile Ser Ser Gly Gly Ser Phe Thr Tyr Tyr Pro Asp Asn Leu
    50                  55                  60

Lys Gly Arg Phe Thr Val Ser Arg Asp Asn Ala Lys Asp Thr Leu Tyr
65                  70                  75                  80

Leu Gln Met Ser Ser Leu Arg Ser Glu Asp Thr Ala Ile Tyr Tyr Cys
                85                  90                  95

Ala Arg Gln Ser Leu Gly Tyr Tyr Phe Asp Ser Trp Gly Gln Gly Thr
            100                 105                 110

Thr Leu Thr Val Ser Ser
        115

<210> 82
<211> 12
<212> PRT
<213> Peptide

<400> 82

Ser Val Pro Trp Trp Thr Gln Ser Leu Leu Met Ser
1               5                   10

<210> 83
<211> 119
<212> PRT
<213> Peptide

<400> 83

Glu Val Gln Leu Gln Gln Ser Gly Pro Val Leu Val Lys Pro Gly Ala
1               5                   10                  15

Ser Val Lys Ile Ser Cys Lys Thr Ser Gly Tyr Thr Phe Thr Glu Tyr
            20                  25                  30

Thr Ile His Trp Met Arg Gln Ser His Gly Lys Ser Leu Glu Trp Ile
        35                  40                  45

Gly Gly Ile Asn Pro Asn Lys Gly Asn Thr Asn Phe Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu His Ser Leu Pro Ser Glu Asp Ser Ala Val Phe Tyr Cys
                85                  90                  95

Ala Arg Ala Asn Trp Asp Val Tyr Ala Val Asp Ser Trp Gly Gln Gly
            100                 105                 110

Thr Ser Val Thr Val Ser Ser
        115

<210> 84
<211> 12
<212> PRT
<213> Peptide

<400> 84

Asp Ile Trp Ser Thr Met Glu His Asn Lys Tyr Asn
1               5                   10

<210> 85
<211> 12
<212> PRT
<213> Peptide

<400> 85

His Ile Asn Ala Ile Gln Tyr Pro Leu Pro His Thr
1               5                   10

<210> 86
<211> 119
<212> PRT
<213> Peptide

<400> 86

Gln Val Gln Leu Gln Gln Pro Gly Ala Glu Leu Val Arg Pro Gly Ala
1 5 10 15

Ser Val Lys Leu Ser Cys Lys Ala Ser Gly Tyr Ser Phe Ala Ser Tyr
20 25 30

Trp Met Asn Trp Val Lys Gln Arg Pro Gly Gln Gly Leu Glu Trp Ile
35 40 45

Gly Met Ile His Pro Ser Asp Ser Gly Thr Ser Leu Asp Glu Lys Phe
50 55 60

Lys Asp Lys Ala Thr Leu Thr Val Asp Lys Ser Ser Asn Thr Ala Tyr
65 70 75 80

Ile Gln Leu Asn Ser Pro Thr Ser Glu Asp Ser Ala Val Tyr Tyr Cys
85 90 95

Ala Arg Glu Gly Tyr Asp Pro Ala Trp Phe Ala Tyr Trp Gly Gln Gly
100 105 110

Thr Leu Val Thr Val Ser Ala
115

<210> 87
<211> 400
<212> PRT
<213> cytokeratin19

<400> 87

Met Thr Ser Tyr Ser Tyr Arg Gln Ser Ser Ala Thr Ser Ser Phe Gly
1               5                   10                  15

Gly Leu Gly Gly Gly Ser Val Arg Phe Gly Pro Gly Val Ala Phe Arg
            20                  25                  30

Ala Pro Ser Ile His Gly Gly Ser Gly Gly Arg Gly Val Ser Val Ser
        35                  40                  45

Ser Ala Arg Phe Val Ser Ser Ser Ser Ser Gly Gly Tyr Gly Gly Gly
    50                  55                  60

Tyr Gly Gly Val Leu Thr Ala Ser Asp Gly Leu Leu Ala Gly Asn Glu
65                  70                  75                  80

Lys Leu Thr Met Gln Asn Leu Asn Asp Arg Leu Ala Ser Tyr Leu Asp
                85                  90                  95

Lys Val Arg Ala Leu Glu Ala Ala Asn Gly Glu Leu Glu Val Lys Ile
            100                 105                 110

Arg Asp Trp Tyr Gln Lys Gln Gly Pro Gly Pro Ser Arg Asp Tyr Ser
115 120 125

His Tyr Tyr Thr Thr Ile Gln Asp Leu Arg Asp Lys Ile Leu Gly Ala
130 135 140

Thr Ile Glu Asn Ser Arg Ile Val Leu Gln Ile Asp Asn Ala Arg Leu
145 150 155 160

Ala Ala Asp Asp Phe Arg Thr Lys Phe Glu Thr Glu Gln Ala Leu Arg
165 170 175

Met Ser Val Glu Ala Asp Ile Asn Gly Leu Arg Arg Val Leu Asp Glu
180 185 190

Leu Thr Leu Ala Arg Thr Asp Leu Glu Met Gln Ile Glu Gly Leu Lys
195 200 205

Glu Glu Leu Ala Tyr Leu Lys Lys Asn His Glu Glu Glu Ile Ser Thr
210 215 220

Leu Arg Gly Gln Val Gly Gly Gln Val Ser Val Glu Val Asp Ser Ala
225 230 235 240

Pro Gly Thr Asp Leu Ala Lys Ile Leu Ser Asp Met Arg Ser Gln Tyr
245 250 255

Glu Val Met Ala Glu Gln Asn Arg Lys Asp Ala Glu Ala Trp Phe Thr
260 265 270

Ser Arg Thr Glu Glu Leu Asn Arg Glu Val Ala Gly His Thr Glu Gln
275 280 285

Leu Gln Met Ser Arg Ser Glu Val Thr Asp Leu Arg Arg Thr Leu Gln
290 295 300

Gly Leu Glu Ile Glu Leu Gln Ser Gln Leu Ser Met Lys Ala Ala Leu
305 310 315 320

Glu Asp Thr Leu Ala Glu Thr Glu Ala Arg Phe Gly Ala Gln Leu Ala
325 330 335

His Ile Gln Ala Leu Ile Ser Gly Ile Glu Ala Gln Leu Gly Asp Val
340 345 350

Arg Ala Asp Ser Glu Arg Gln Asn Gln Glu Tyr Gln Arg Leu Met Asp
355 360 365

Ile Lys Ser Arg Leu Glu Gln Glu Ile Ala Thr Tyr Arg Ser Leu Leu
370 375 380

Glu Gly Gln Glu Asp His Tyr Asn Asn Leu Ser Ala Ser Lys Val Leu
385 390 395 400

<210> 88
<211> 112
<212> PRT
<213> Peptide

<400> 88

Asp Val Gln Leu Gln Glu Ser Gly Pro Gly Leu Val Lys Pro Ser Gln
1 5 10 15

Ser Leu Ser Leu Thr Cys Thr Val Thr Gly Tyr Ser Ile Ile Asn Asp
20 25 30

Tyr Ala Trp Asn Trp Ile Arg Gln Phe Pro Gly Asn Lys Leu Glu Trp
35 40 45

Met Ala Tyr Ile Ser Tyr Gly Gly Ser Ile Gly Tyr Lys Pro Ser Leu
50 55 60

Lys Ser Arg Ile Ser Ile Thr Arg Asp Thr Ser Lys Asn Gln Phe Phe
65 70 75 80

Leu Gln Leu Asn Ser Val Thr Thr Glu Asp Thr Ala Thr Tyr Tyr Cys
85 90 95

Ala Arg Gly Gly Phe Tyr Ala Leu Asp Tyr Trp Gly Gln Gly Thr Ser
100 105 110

<210> 89
<211> 118
<212> PRT
<213> Peptide

<400> 89

Glu Val Gln Leu Gln Glu Ser Gly Pro Ser Leu Val Lys Pro Ser Gln
1               5                   10                  15

Thr Leu Ser Leu Thr Cys Ser Val Thr Gly Asp Ser Ile Thr Ser Gly
            20                  25                  30

Ser Trp Asn Trp Ile Arg Glu Phe Pro Gly Asn Lys Leu Glu Tyr Met
        35                  40                  45

Gly Tyr Ile Ser Tyr Ser Gly Ser Thr Asp Tyr Ser Pro Ser Leu Lys
    50                  55                  60

Ser Arg Ile Ser Ile Thr Arg Asp Thr Ser Lys Asn Gln Tyr Tyr Leu
65                  70                  75                  80

Gln Leu Asn Ser Val Thr Thr Glu Asp Thr Ala Thr Tyr Tyr Cys Ala
                85                  90                  95

Thr His Tyr Tyr Gly Tyr Leu Ser Leu Asp Tyr Trp Gly Gln Gly Thr
            100                 105                 110

Ser Val Thr Val Ser Ser
        115

<210> 90
<211> 114
<212> PRT
<213> Peptide

<400> 90

Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Ser Trp Val Arg Gln Ala Pro Gly Lys Gly Leu Glu Trp Ile
        35                  40                  45

Ser Arg Ile Arg Ser Lys Ser Asn Asn Tyr Ala Thr Tyr Tyr Val Asp
    50                  55                  60

Ser Val Lys Asp Arg Phe Thr Ile Tyr Arg Asp Asp Ser Gln Ser Met
65                  70                  75                  80

Leu Tyr Leu Gln Met Asn Asn Leu Lys Thr Glu Asp Thr Ala Ile Tyr
                85                  90                  95

Tyr Cys Val Arg Glu Gly Asp Trp Gly Gln Gly Thr Leu Val Thr Val
            100                 105                 110

Ser Ala

<210> 91
<211> 114
<212> PRT
<213> Peptide

<400> 91

Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Ser Thr Ser
20 25 30

Ala Met Asn Trp Val Arg Gln Ala Pro Gly Lys Gly Leu Glu Trp Ile
35 40 45

Ser Arg Ile Arg Ser Lys Thr Asn Asn Tyr Ala Thr Tyr Tyr Val Asp
50 55 60

Ser Val Lys Asp Arg Phe Thr Ile Tyr Arg Asp Asp Ser Gln Asn Met
65 70 75 80

Leu Tyr Leu Gln Met Asn Asn Leu Lys Thr Glu Asp Thr Ala Met Tyr
85 90 95

Tyr Cys Val Arg Glu Gly Asp Trp Gly Gln Gly Thr Leu Val Thr Val
100 105 110

Ser Ala

<210> 92
<211> 122
<212> PRT
<213> Peptide

<400> 92

Gln Ile Gln Leu Val Gln Ser Gly Pro Glu Leu Lys Lys Pro Gly Glu
1 5 10 15

Thr Val Glu Ile Ser Cys Lys Ala Ser Gly Tyr Thr Phe Thr Asp Tyr
20 25 30

Ser Met His Trp Val Lys Gln Ala Pro Gly Lys Gly Leu Lys Trp Met
35 40 45

Gly Trp Ile Asn Thr Glu Thr Gly Glu Pro Thr Tyr Ala Asp Asp Phe
50 55 60

Lys Gly Arg Phe Ala Phe Ser Leu Glu Thr Ser Ala Thr Thr Ala Tyr
65 70 75 80

Leu Gln Ile Asn Asn Leu Lys Asn Glu Asp Thr Ala Thr Tyr Phe Cys
85 90 95

Ala Arg Gly Gly Tyr Tyr Gly Asn Tyr Asp Tyr Ala Met Asp Tyr Trp
100 105 110

Gly Gln Gly Thr Ser Leu Thr Val Ser Ser
115 120

<210> 93
<211> 15
<212> PRT
<213> Peptide

<400> 93

Asp Val Arg Ala Asp Ser Glu Arg Gln Asn Gln Glu Tyr Gln Arg
1 5 10 15

<210> 94
<211> 15
<212> PRT
<213> Peptide

<400> 94

Ser Glu Arg Gln Asn Gln Glu Tyr Gln Arg Leu Met Asp Ile Lys
1 5 10 15

<210> 95
<211> 15
<212> PRT
<213> Peptide

<400> 95

Gln Glu Tyr Gln Arg Leu Met Asp Ile Lys Ser Arg Leu Glu Gln
1 5 10 15

<210> 96
<211> 15
<212> PRT
<213> Peptide

<400> 96

Met Lys Ala Ala Leu Glu Asp Thr Leu Ala Glu Thr Glu Ala Arg
1 5 10 15

SEQUENCE LISTING

<110> Biosystems International

<120> Combinatorial biomarkers for clinical applications in lung cancer patient management

<130> B1123EPPC

<160> 96

<170> PatentIn version 3.3

<210> 1
<211> 12
<212> PRT
<213> Artificial

<220>
<223> Binding peptide recognized by antibody

<400> 1

```
Asn Asn Ser Tyr Leu Asp Glu Glu Gly Thr Trp Leu
1               5                   10
```

<210> 2
<211> 12
<212> PRT
<213> Artificial

<220>
<223> Binding peptide recognized by antibody

<400> 2

```
Phe Pro Thr His Asn Pro Thr Ser Pro Leu Asp Met
1               5                   10
```

<210> 3
<211> 12
<212> PRT
<213> Artificial

<220>
<223> Binding peptide recognized by antibody

<400> 3

```
Asp Leu Asn Asp Pro Pro Phe Phe Thr Ser Val Ser
1               5                   10
```

<210> 4
<211> 12
<212> PRT
<213> Artificial

<220>
<223> Binding peptide recognized by antibody

<400> 4

```
Thr Thr His Trp Asp His Pro Phe Tyr Asp Asn Thr
1               5                   10
```

<210> 5
<211> 12
<212> PRT
<213> Artificial

<220>
<223> Binding peptide recognized by antibody

<400> 5

Ala Trp His Pro Ser Ser Pro Leu Asp Tyr Gly Phe
1 5 10

<210> 6
<211> 12
<212> PRT
<213> Artificial

<220>
<223> Binding peptide recognized by antibody

<400> 6

Ala Asn Ser Val Val Tyr Lys Pro Ser Ser Pro Leu
1 5 10

<210> 7
<211> 12
<212> PRT
<213> Artificial

<220>
<223> Binding peptide recognized by antibody

<400> 7

Thr Ser Tyr Asn Thr Asp His Pro Phe His Tyr Pro
1 5 10

<210> 8
<211> 12
<212> PRT
<213> Artificial

<220>
<223> Binding peptide recognized by antibody

<400> 8

Ser Pro Ser Ala Pro Ser Ser Pro Leu Asp Ser Met
1 5 10

<210> 9
<211> 12
<212> PRT
<213> Artificial

<220>
<223> Binding peptide recognized by antibody

<400> 9

Val Arg Ala Cys Val Asp Cys Asp Gln Pro Phe Tyr
1 5 10

<210> 10
<211> 12
<212> PRT
<213> Artificial

<220>
<223> Binding peptide recognized by antibody

<400> 10

Ile Ala Pro Ser Ser Phe Leu Asp Glu Arg Thr Asp
1 5 10

<210> 11
<211> 12
<212> PRT
<213> Artificial

<220>
<223> Binding peptide recognized by antibody

<400> 11

Thr Arg Pro His Thr Asp Pro Pro Phe Trp Trp Ala
1 5 10

<210> 12
<211> 12
<212> PRT
<213> Artificial

<220>
<223> Binding peptide recognized by antibody

<400> 12

Glu Pro Asp Pro Pro Phe Tyr Thr His Leu Thr Ala
1 5 10

<210> 13
<211> 12
<212> PRT
<213> Artificial

<220>
<223> Binding peptide recognized by antibody

<400> 13

Ser Gln Met Lys Pro Ser Ser His Leu Asp Trp Asp
1 5 10

<210> 14
<211> 12
<212> PRT

<213> Artificial

<220>
<223> Binding peptide recognized by antibody

<400> 14

```
His Thr Leu Pro Gln Phe Leu Glu Trp His Lys Arg
1               5                   10
```

<210> 15
<211> 12
<212> PRT
<213> Artificial

<220>
<223> Binding peptide recognized by antibody

<400> 15

```
Gly Gln Val Val Phe Gln Asp Trp Leu Leu Val Arg
1               5                   10
```

<210> 16
<211> 12
<212> PRT
<213> Artificial

<220>
<223> Binding peptide recognized by antibody

<400> 16

```
His Glu Asn Ala Asn Lys Ile Ala Pro Trp Val Gln
1               5                   10
```

<210> 17
<211> 12
<212> PRT
<213> Artificial

<220>
<223> Binding peptide recognized by antibody

<400> 17

```
Gln Leu Lys Ile Ala Pro Trp Val Ile Glu Ser Pro
1               5                   10
```

<210> 18
<211> 12
<212> PRT
<213> Artificial

<220>
<223> Binding peptide recognized by antibody

<400> 18

Thr Asp Thr Leu Asp Ser Phe Leu Gly Arg Ser Pro
1 5 10

<210> 19
<211> 12
<212> PRT
<213> Artificial

<220>
<223> Binding peptide recognized by antibody

<400> 19

Lys Pro Pro Arg Gln Val Val Ala Pro Trp Val Met
1 5 10

<210> 20
<211> 12
<212> PRT
<213> Artificial

<220>
<223> Binding peptide recognized by antibody

<400> 20

Ile Ala Gln Thr Leu Pro Ala Phe Leu Ala Thr Arg
1 5 10

<210> 21
<211> 12
<212> PRT
<213> Artificial

<220>
<223> Binding peptide recognized by antibody

<400> 21

Thr Glu Thr Leu His Glu Phe Leu Asp Gly Arg Lys
1 5 10

<210> 22
<211> 12
<212> PRT
<213> Artificial

<220>
<223> Binding peptide recognized by antibody

<400> 22

Lys His Leu Glu His Pro Thr Tyr His Leu Trp His
1 5 10

<210> 23
<211> 12
<212> PRT

<213> Artificial

<220>
<223> Binding peptide recognized by antibody

<400> 23

```
Ser Leu Asn Thr Ile Ala Pro Trp Ile Leu Thr Ala
1               5                   10
```

<210> 24
<211> 12
<212> PRT
<213> Artificial

<220>
<223> Binding peptide recognized by antibody

<400> 24

```
Lys Ala Thr Thr His Asn Ile Ala Trp Trp Val Ala
1               5                   10
```

<210> 25
<211> 12
<212> PRT
<213> Artificial

<220>
<223> Binding peptide recognized by antibody

<400> 25

```
His Asp Asn Ile Asn Met Ala Trp Trp Val Glu Trp
1               5                   10
```

<210> 26
<211> 12
<212> PRT
<213> Artificial

<220>
<223> Binding peptide recognized by antibody

<400> 26

```
Glu Trp Tyr Val Ser Thr Ser Leu Leu Pro Leu Pro
1               5                   10
```

<210> 27
<211> 12
<212> PRT
<213> Artificial

<220>
<223> Binding peptide recognized by antibody

<400> 27

His Ile Tyr Tyr Ile Ser Glu Ser Leu Leu Pro Met
1 5 10

<210> 28
<211> 12
<212> PRT
<213> Artificial

<220>
<223> Binding peptide recognized by antibody

<400> 28

His Val Glu Tyr Glu Ile Ile Glu Gln Met Ile Tyr
1 5 10

<210> 29
<211> 12
<212> PRT
<213> Artificial

<220>
<223> Binding peptide recognized by antibody

<400> 29

Ser Pro Phe Phe Tyr Thr Leu Thr Arg Val Pro Met
1 5 10

<210> 30
<211> 12
<212> PRT
<213> Artificial

<220>
<223> Binding peptide recognized by antibody

<400> 30

Ser Ser Tyr Leu Tyr Thr Thr Gln Phe Val Pro Leu
1 5 10

<210> 31
<211> 12
<212> PRT
<213> Artificial

<220>
<223> Binding peptide recognized by antibody

<400> 31

Thr Gly Trp Met Ile Ser Thr Thr Leu Ile Pro Thr
1 5 10

<210> 32
<211> 12
<212> PRT

<213> Artificial

<220>
<223> Binding peptide recognized by antibody

<400> 32

```
Tyr Lys Tyr Ser Tyr Thr Thr Glu Leu Ile Leu Ile
1               5                   10
```

<210> 33
<211> 12
<212> PRT
<213> Artificial

<220>
<223> Binding peptide recognized by antibody

<400> 33

```
Ser Tyr Thr Tyr Thr Thr His Leu Leu Pro Leu Ala
1               5                   10
```

<210> 34
<211> 12
<212> PRT
<213> Artificial

<220>
<223> Binding peptide recognized by antibody

<400> 34

```
Thr Met Thr Gln Tyr Tyr Tyr Ser Thr Thr His Tyr
1               5                   10
```

<210> 35
<211> 12
<212> PRT
<213> Artificial

<220>
<223> Binding peptide recognized by antibody

<400> 35

```
Gln Phe Trp Thr Ile Thr Thr Thr Leu Val Pro His
1               5                   10
```

<210> 36
<211> 12
<212> PRT
<213> Artificial

<220>
<223> Binding peptide recognized by antibody

<400> 36

Gly Pro Lys Met Asp Ala Leu Ser Lys Ala Glu Asn
1 5 10

<210> 37
<211> 12
<212> PRT
<213> Artificial

<220>
<223> Binding peptide recognized by antibody

<400> 37

Gly Pro Arg Trp Leu Asp Ala Leu Phe Met Ala Glu
1 5 10

<210> 38
<211> 12
<212> PRT
<213> Artificial

<220>
<223> Binding peptide recognized by antibody

<400> 38

His Ile Gly Ala His Asp Ser Phe Glu Thr Lys His
1 5 10

<210> 39
<211> 12
<212> PRT
<213> Artificial

<220>
<223> Binding peptide recognized by antibody

<400> 39

Asn Asn Ser Tyr Leu Asp Glu Glu Gly Thr Trp Leu
1 5 10

<210> 40
<211> 12
<212> PRT
<213> Artificial

<220>
<223> Binding peptide recognized by antibody

<400> 40

Ala Met Asn Thr Val Val Cys Tyr Asp Glu Pro Cys
1 5 10

<210> 41
<211> 12
<212> PRT

<213> Artificial

<220>
<223> Binding peptide recognized by antibody

<400> 41

```
Thr Asn Ser Pro Leu Asp Glu Leu Gly Thr His Pro
1               5                   10
```

<210> 42
<211> 12
<212> PRT
<213> Artificial

<220>
<223> Binding peptide recognized by antibody

<400> 42

```
Ser Thr Asn Ser His Glu His Trp Pro Met Ser Pro
1               5                   10
```

<210> 43
<211> 12
<212> PRT
<213> Artificial

<220>
<223> Binding peptide recognized by antibody

<400> 43

```
Asn His Phe Ser Ser Leu Gly Ile Leu Thr Ala Ala
1               5                   10
```

<210> 44
<211> 12
<212> PRT
<213> Artificial

<220>
<223> Binding peptide recognized by antibody

<400> 44

```
Ile Pro Gln Pro Ser Ile Leu Thr Thr Pro Met Leu
1               5                   10
```

<210> 45
<211> 12
<212> PRT
<213> Artificial

<220>
<223> Binding peptide recognized by antibody

<400> 45

Leu Arg Leu Pro Asn Thr Tyr Glu Asn Ala Lys Asn
1 5 10

<210> 46
<211> 12
<212> PRT
<213> Artificial

<220>
<223> Binding peptide recognized by antibody

<400> 46

Gly Pro Leu Ser Pro Ala His Leu Ser Gln Arg Asn
1 5 10

<210> 47
<211> 12
<212> PRT
<213> Artificial

<220>
<223> Binding peptide recognized by antibody

<400> 47

Phe Pro Leu Pro Phe Asp Pro Glu Met Leu Pro Leu
1 5 10

<210> 48
<211> 12
<212> PRT
<213> Artificial

<220>
<223> Binding peptide recognized by antibody

<400> 48

Phe Pro Leu Pro Pro Glu Tyr Ser Met Lys Lys Asp
1 5 10

<210> 49
<211> 12
<212> PRT
<213> Artificial

<220>
<223> Binding peptide recognized by antibody

<400> 49

Phe Lys Ile Pro Glu Gly Asn Asn Val Ser Val Leu
1 5 10

<210> 50
<211> 12
<212> PRT

<213> Artificial

<220>
<223> Binding peptide recognized by antibody

<400> 50

```
Phe Arg Leu Pro Pro His Asp Ser Ser Asp His His
1               5                   10
```

<210> 51
<211> 12
<212> PRT
<213> Artificial

<220>
<223> Binding peptide recognized by antibody

<400> 51

```
Phe Phe Leu Pro His Ser Ala Thr Leu Pro Ser His
1               5                   10
```

<210> 52
<211> 12
<212> PRT
<213> Artificial

<220>
<223> Binding peptide recognized by antibody

<400> 52

```
Phe Ile Leu Pro His Arg Asn Gly Tyr Ile Asp Thr
1               5                   10
```

<210> 53
<211> 12
<212> PRT
<213> Artificial

<220>
<223> Binding peptide recognized by antibody

<400> 53

```
Leu Lys Leu Pro Asn Pro Asp Thr Ala Ala Pro Thr
1               5                   10
```

<210> 54
<211> 12
<212> PRT
<213> Artificial

<220>
<223> Binding peptide recognized by antibody

<400> 54

Asn Asn Asn Met Gly Asn Ile Leu Leu Ile Thr Pro
1 5 10

<210> 55
<211> 12
<212> PRT
<213> Artificial

<220>
<223> Binding peptide recognized by antibody

<400> 55

Val Leu Leu Val Pro His His Ser His Gln Tyr Asp
1 5 10

<210> 56
<211> 12
<212> PRT
<213> Artificial

<220>
<223> Binding peptide recognized by antibody

<400> 56

Tyr Leu Lys Glu Ser Thr His Val Leu Leu Ala Ala
1 5 10

<210> 57
<211> 12
<212> PRT
<213> Artificial

<220>
<223> Binding peptide recognized by antibody

<400> 57

His Asp Asp Trp Tyr Ile Ser Thr Ser His Thr Asp
1 5 10

<210> 58
<211> 12
<212> PRT
<213> Artificial

<220>
<223> Binding peptide recognized by antibody

<400> 58

Ser Tyr Thr Tyr Thr Thr His Leu Leu Pro Leu Ala
1 5 10

<210> 59
<211> 12
<212> PRT

<213> Artificial

<220>
<223> Binding peptide recognized by antibody

<400> 59

Glu Trp Tyr Val Tyr Glu Lys Leu Phe Pro Leu Pro
1 5 10

<210> 60
<211> 12
<212> PRT
<213> Artificial

<220>
<223> Binding peptide recognized by antibody

<400> 60

Tyr Lys Tyr Ser Tyr Thr Thr Glu Leu Ile Leu Ile
1 5 10

<210> 61
<211> 12
<212> PRT
<213> Artificial

<220>
<223> Binding peptide recognized by antibody

<400> 61

His Gln Tyr Tyr Tyr Thr Ser Ser Leu Ile Tyr Thr
1 5 10

<210> 62
<211> 12
<212> PRT
<213> Artificial

<220>
<223> Binding peptide recognized by antibody

<400> 62

Thr Ala Trp His Thr Thr Glu Glu Leu Ile Met Leu
1 5 10

<210> 63
<211> 12
<212> PRT
<213> Artificial

<220>
<223> Binding peptide recognized by antibody

<400> 63

Asn Gly Tyr Ile Phe Thr Thr Gln Leu Ile Trp Ala
1               5                   10

<210> 64
<211> 12
<212> PRT
<213> Artificial

<220>
<223> Binding peptide recognized by antibody

<400> 64

Thr Val Asn Pro Leu Leu Arg Leu Leu Ser Met Gly
1               5                   10

<210> 65
<211> 12
<212> PRT
<213> Artificial

<220>
<223> Binding peptide recognized by antibody

<400> 65

Asn Asn Pro Met Leu Val Leu Leu Ser His Gly Ala
1               5                   10

<210> 66
<211> 12
<212> PRT
<213> Artificial

<220>
<223> Binding peptide recognized by antibody

<400> 66

Thr Val Pro Gln Pro Gln Arg Leu Thr Gln Lys Ser
1               5                   10

<210> 67
<211> 12
<212> PRT
<213> Artificial

<220>
<223> Binding peptide recognized by antibody

<400> 67

Ala Lys Tyr Glu Ser Leu Leu Arg Met Leu Ala Ala
1               5                   10

<210> 68
<211> 12
<212> PRT

<213> Artificial

<220>
<223> Binding peptide recognized by antibody

<400> 68

```
Gly His Glu Ser Gly Tyr His Ile Ser Glu Arg Ala
1               5                   10
```

<210> 69
<211> 12
<212> PRT
<213> Artificial

<220>
<223> Binding peptide recognized by antibody

<400> 69

```
Gly His Glu Glu His Leu Trp Gln Ile Leu His Phe
1               5                   10
```

<210> 70
<211> 12
<212> PRT
<213> Artificial

<220>
<223> Binding peptide recognized by antibody

<400> 70

```
Gly His Glu Glu Gln Arg Asn Trp Ser Met Glu Gly
1               5                   10
```

<210> 71
<211> 12
<212> PRT
<213> Artificial

<220>
<223> Binding peptide recognized by antibody

<400> 71

```
Ser Thr Tyr Thr Asp Met Leu Ala Asp Leu Ser Ala
1               5                   10
```

<210> 72
<211> 12
<212> PRT
<213> Artificial

<220>
<223> Binding peptide recognized by antibody

<400> 72

Ser Tyr Glu Ser Leu Leu Met Lys Leu Ala Arg Gly
1 5 10

<210> 73
<211> 12
<212> PRT
<213> Artificial

<220>
<223> Binding peptide recognized by antibody

<400> 73

Tyr Arg Asp Ser Pro Gly Pro Arg Asp Lys Leu Leu
1 5 10

<210> 74
<211> 12
<212> PRT
<213> Artificial

<220>
<223> Binding peptide recognized by antibody

<400> 74

Gly His Glu Glu Arg Glu Tyr Gln Met Met Leu Gln
1 5 10

<210> 75
<211> 12
<212> PRT
<213> Artificial

<220>
<223> Binding peptide recognized by antibody

<400> 75

Cys Leu Pro Tyr Pro Leu Cys Arg Gly Thr Ala Thr
1 5 10

<210> 76
<211> 12
<212> PRT
<213> Artificial

<220>
<223> Binding peptide recognized by antibody

<400> 76

Thr Glu Cys Leu Pro Tyr Pro Leu Cys Leu Tyr Asn
1 5 10

<210> 77
<211> 117
<212> PRT

<213> Artificial

<220>
<223> Antibody heavy chain variable region

<400> 77

```
Glu Val Gln Leu Gln Gln Ser Gly Ala Asp Leu Val Lys Pro Gly Ala
1               5                   10                  15

Ser Val Lys Leu Ser Cys Thr Ala Ser Gly Phe Asn Ile Lys Asp Thr
            20                  25                  30

Tyr Met His Trp Met Lys Gln Arg Pro Glu Gln Gly Leu Glu Trp Ile
        35                  40                  45

Gly Arg Ile Asp Pro Ala Asn Gly Asn Ser Lys Tyr Asp Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Phe Cys
                85                  90                  95

Thr Lys Ser Ala Gly Val Pro Phe Ala Tyr Trp Gly Gln Gly Thr Leu
            100                 105                 110

Val Thr Val Ser Ala
        115
```

<210> 78
<211> 12
<212> PRT
<213> Artificial

<220>
<223> Binding peptide recognized by antibody

<400> 78

```
Asn Glu Thr Trp Ser Glu Phe Trp Arg Leu His Asn
1               5                   10
```

<210> 79
<211> 12
<212> PRT
<213> Artificial

<220>
<223> Binding peptide recognized by antibody

<400> 79

Thr Ala Gln Asp Asp Arg Ile Phe His Met Met His
1               5                   10

<210> 80
<211> 12
<212> PRT
<213> Artificial

<220>
<223> Binding peptide recognized by antibody

<400> 80

Arg Gly Thr Gln Asp Met Glu Tyr Phe Ala Met His
1               5                   10

<210> 81
<211> 118
<212> PRT
<213> Artificial

<220>
<223> Antibody heavy chain variable region

<400> 81

Glu Val Met Leu Val Glu Ser Gly Gly Gly Leu Val Lys Pro Gly Gly
1               5                   10                  15

Ser Leu Lys Leu Ser Cys Ala Ala Ser Glu Phe Thr Phe Ser Asn Tyr
            20                  25                  30

Ala Met Ser Trp Val Arg Gln Thr Pro Glu Lys Arg Leu Glu Trp Val
        35                  40                  45

Ala Thr Ile Ser Ser Gly Gly Ser Phe Thr Tyr Tyr Pro Asp Asn Leu
    50                  55                  60

Lys Gly Arg Phe Thr Val Ser Arg Asp Asn Ala Lys Asp Thr Leu Tyr
65                  70                  75                  80

Leu Gln Met Ser Ser Leu Arg Ser Glu Asp Thr Ala Ile Tyr Tyr Cys
                85                  90                  95

Ala Arg Gln Ser Leu Gly Tyr Tyr Phe Asp Ser Trp Gly Gln Gly Thr
            100                 105                 110

Thr Leu Thr Val Ser Ser
        115

<210> 82

<211> 12
<212> PRT
<213> Artificial

<220>
<223> Binding peptide recognized by antibody

<400> 82

```
Ser Val Pro Trp Trp Thr Gln Ser Leu Leu Met Ser
1               5                   10
```

<210> 83
<211> 119
<212> PRT
<213> Artificial

<220>
<223> Antibody heavy chain variable region

<400> 83

```
Glu Val Gln Leu Gln Gln Ser Gly Pro Val Leu Val Lys Pro Gly Ala
1               5                   10                  15

Ser Val Lys Ile Ser Cys Lys Thr Ser Gly Tyr Thr Phe Thr Glu Tyr
            20                  25                  30

Thr Ile His Trp Met Arg Gln Ser His Gly Lys Ser Leu Glu Trp Ile
        35                  40                  45

Gly Gly Ile Asn Pro Asn Lys Gly Asn Thr Asn Phe Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu His Ser Leu Pro Ser Glu Asp Ser Ala Val Phe Tyr Cys
                85                  90                  95

Ala Arg Ala Asn Trp Asp Val Tyr Ala Val Asp Ser Trp Gly Gln Gly
            100                 105                 110

Thr Ser Val Thr Val Ser Ser
        115
```

<210> 84
<211> 12
<212> PRT
<213> Artificial

<220>

<223> Binding peptide recognized by antibody

<400> 84

```
Asp Ile Trp Ser Thr Met Glu His Asn Lys Tyr Asn
1               5                   10
```

<210> 85
<211> 12
<212> PRT
<213> Artificial

<220>
<223> Binding peptide recognized by antibody

<400> 85

```
His Ile Asn Ala Ile Gln Tyr Pro Leu Pro His Thr
1               5                   10
```

<210> 86
<211> 119
<212> PRT
<213> Artificial

<220>
<223> Antibody heavy chain variable region

<400> 86

```
Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Ser Phe Ala Ser Tyr
            20                  25                  30

Trp Met Asn Trp Val Lys Gln Arg Pro Gly Gln Gly Leu Glu Trp Ile
        35                  40                  45

Gly Met Ile His Pro Ser Asp Ser Gly Thr Ser Leu Asp Glu Lys Phe
    50                  55                  60

Lys Asp Lys Ala Thr Leu Thr Val Asp Lys Ser Ser Asn Thr Ala Tyr
65                  70                  75                  80

Ile Gln Leu Asn Ser Pro Thr Ser Glu Asp Ser Ala Val Tyr Tyr Cys
                85                  90                  95

Ala Arg Glu Gly Tyr Asp Pro Ala Trp Phe Ala Tyr Trp Gly Gln Gly
            100                 105                 110

Thr Leu Val Thr Val Ser Ala
        115
```

<210> 87
<211> 400
<212> PRT
<213> Artificial

<220>
<223> CYFRA protein

<400> 87

```
Met Thr Ser Tyr Ser Tyr Arg Gln Ser Ser Ala Thr Ser Ser Phe Gly
1               5                   10                  15

Gly Leu Gly Gly Gly Ser Val Arg Phe Gly Pro Gly Val Ala Phe Arg
            20                  25                  30

Ala Pro Ser Ile His Gly Gly Ser Gly Gly Arg Gly Val Ser Val Ser
        35                  40                  45

Ser Ala Arg Phe Val Ser Ser Ser Ser Ser Gly Gly Tyr Gly Gly Gly
    50                  55                  60

Tyr Gly Gly Val Leu Thr Ala Ser Asp Gly Leu Leu Ala Gly Asn Glu
65                  70                  75                  80

Lys Leu Thr Met Gln Asn Leu Asn Asp Arg Leu Ala Ser Tyr Leu Asp
                85                  90                  95

Lys Val Arg Ala Leu Glu Ala Ala Asn Gly Glu Leu Glu Val Lys Ile
            100                 105                 110

Arg Asp Trp Tyr Gln Lys Gln Gly Pro Gly Pro Ser Arg Asp Tyr Ser
        115                 120                 125

His Tyr Tyr Thr Thr Ile Gln Asp Leu Arg Asp Lys Ile Leu Gly Ala
    130                 135                 140

Thr Ile Glu Asn Ser Arg Ile Val Leu Gln Ile Asp Asn Ala Arg Leu
145                 150                 155                 160

Ala Ala Asp Asp Phe Arg Thr Lys Phe Glu Thr Glu Gln Ala Leu Arg
                165                 170                 175

Met Ser Val Glu Ala Asp Ile Asn Gly Leu Arg Arg Val Leu Asp Glu
```

```
                180                 185                 190

Leu Thr Leu Ala Arg Thr Asp Leu Glu Met Gln Ile Glu Gly Leu Lys
        195                 200                 205

Glu Glu Leu Ala Tyr Leu Lys Lys Asn His Glu Glu Glu Ile Ser Thr
    210                 215                 220

Leu Arg Gly Gln Val Gly Gly Gln Val Ser Val Glu Val Asp Ser Ala
225                 230                 235                 240

Pro Gly Thr Asp Leu Ala Lys Ile Leu Ser Asp Met Arg Ser Gln Tyr
                245                 250                 255

Glu Val Met Ala Glu Gln Asn Arg Lys Asp Ala Glu Ala Trp Phe Thr
            260                 265                 270

Ser Arg Thr Glu Glu Leu Asn Arg Glu Val Ala Gly His Thr Glu Gln
        275                 280                 285

Leu Gln Met Ser Arg Ser Glu Val Thr Asp Leu Arg Arg Thr Leu Gln
    290                 295                 300

Gly Leu Glu Ile Glu Leu Gln Ser Gln Leu Ser Met Lys Ala Ala Leu
305                 310                 315                 320

Glu Asp Thr Leu Ala Glu Thr Glu Ala Arg Phe Gly Ala Gln Leu Ala
                325                 330                 335

His Ile Gln Ala Leu Ile Ser Gly Ile Glu Ala Gln Leu Gly Asp Val
            340                 345                 350

Arg Ala Asp Ser Glu Arg Gln Asn Gln Glu Tyr Gln Arg Leu Met Asp
        355                 360                 365

Ile Lys Ser Arg Leu Glu Gln Glu Ile Ala Thr Tyr Arg Ser Leu Leu
    370                 375                 380

Glu Gly Gln Glu Asp His Tyr Asn Asn Leu Ser Ala Ser Lys Val Leu
385                 390                 395                 400
```

<210> 88
<211> 112
<212> PRT
<213> Artificial

<220>
<223> Antibody heavy chain variable region

<400> 88

```
Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Ile Asn Asp
            20                  25                  30

Tyr Ala Trp Asn Trp Ile Arg Gln Phe Pro Gly Asn Lys Leu Glu Trp
        35                  40                  45

Met Ala Tyr Ile Ser Tyr Gly Gly Ser Ile Gly Tyr Lys Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Arg Gly Gly Phe Tyr Ala Leu Asp Tyr Trp Gly Gln Gly Thr Ser
            100                 105                 110
```

<210> 89
<211> 118
<212> PRT
<213> Artificial

<220>
<223> Antibody heavy chain variable region

<400> 89

Glu Val Gln Leu Gln Glu Ser Gly Pro Ser Leu Val Lys Pro Ser Gln
1               5                   10                  15

Thr Leu Ser Leu Thr Cys Ser Val Thr Gly Asp Ser Ile Thr Ser Gly
            20                  25                  30

Ser Trp Asn Trp Ile Arg Glu Phe Pro Gly Asn Lys Leu Glu Tyr Met
        35                  40                  45

Gly Tyr Ile Ser Tyr Ser Gly Ser Thr Asp Tyr Ser Pro Ser Leu Lys
    50                  55                  60

Ser Arg Ile Ser Ile Thr Arg Asp Thr Ser Lys Asn Gln Tyr Tyr Leu
65                  70                  75                  80

Gln Leu Asn Ser Val Thr Thr Glu Asp Thr Ala Thr Tyr Tyr Cys Ala
                85                  90                  95

Thr His Tyr Tyr Gly Tyr Leu Ser Leu Asp Tyr Trp Gly Gln Gly Thr
            100                 105                 110

Ser Val Thr Val Ser Ser
        115

<210> 90
<211> 114
<212> PRT
<213> Artificial

<220>
<223> Antibody heavy chain variable region

<400> 90

Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Ser Trp Val Arg Gln Ala Pro Gly Lys Gly Leu Glu Trp Ile
        35                  40                  45

Ser Arg Ile Arg Ser Lys Ser Asn Asn Tyr Ala Thr Tyr Tyr Val Asp
    50                  55                  60

Ser Val Lys Asp Arg Phe Thr Ile Tyr Arg Asp Asp Ser Gln Ser Met
65                  70                  75                  80

Leu Tyr Leu Gln Met Asn Asn Leu Lys Thr Glu Asp Thr Ala Ile Tyr
                85                  90                  95

Tyr Cys Val Arg Glu Gly Asp Trp Gly Gln Gly Thr Leu Val Thr Val
            100                 105                 110

Ser Ala

<210> 91
<211> 114
<212> PRT
<213> Artificial

<220>
<223> Antibody heavy chain variable region

<400> 91

Glu Val Gln Leu Val Glu Ser Gly Gly Gly Leu Val Gln Pro Lys Gly
1               5                   10                  15

Ser Leu Lys Leu Ser Cys Ala Ala Ser Gly Phe Thr Phe Ser Thr Ser
20 25 30

Ala Met Asn Trp Val Arg Gln Ala Pro Gly Lys Gly Leu Glu Trp Ile
35 40 45

Ser Arg Ile Arg Ser Lys Thr Asn Asn Tyr Ala Thr Tyr Tyr Val Asp
50 55 60

Ser Val Lys Asp Arg Phe Thr Ile Tyr Arg Asp Asp Ser Gln Asn Met
65 70 75 80

Leu Tyr Leu Gln Met Asn Asn Leu Lys Thr Glu Asp Thr Ala Met Tyr
85 90 95

Tyr Cys Val Arg Glu Gly Asp Trp Gly Gln Gly Thr Leu Val Thr Val
100 105 110

Ser Ala

<210> 92
<211> 122
<212> PRT
<213> Artificial

<220>
<223> Antibody heavy chain variable region

<400> 92

Gln Ile Gln Leu Val Gln Ser Gly Pro Glu Leu Lys Lys Pro Gly Glu
1 5 10 15

Thr Val Glu Ile Ser Cys Lys Ala Ser Gly Tyr Thr Phe Thr Asp Tyr
20 25 30

Ser Met His Trp Val Lys Gln Ala Pro Gly Lys Gly Leu Lys Trp Met
35 40 45

Gly Trp Ile Asn Thr Glu Thr Gly Glu Pro Thr Tyr Ala Asp Asp Phe
50 55 60

Lys Gly Arg Phe Ala Phe Ser Leu Glu Thr Ser Ala Thr Thr Ala Tyr
65 70 75 80

Leu Gln Ile Asn Asn Leu Lys Asn Glu Asp Thr Ala Thr Tyr Phe Cys
85 90 95

Ala Arg Gly Gly Tyr Tyr Gly Asn Tyr Asp Tyr Ala Met Asp Tyr Trp
100 105 110

Gly Gln Gly Thr Ser Leu Thr Val Ser Ser
115 120

<210> 93
<211> 15
<212> PRT
<213> Artificial

<220>
<223> CYFRA protein

<400> 93

Asp Val Arg Ala Asp Ser Glu Arg Gln Asn Gln Glu Tyr Gln Arg
1 5 10 15

<210> 94
<211> 15
<212> PRT
<213> Artificial

<220>
<223> CYFRA protein

<400> 94

Ser Glu Arg Gln Asn Gln Glu Tyr Gln Arg Leu Met Asp Ile Lys
1 5 10 15

<210> 95
<211> 15
<212> PRT
<213> Artificial

<220>
<223> CYFRA protein

<400> 95

Gln Glu Tyr Gln Arg Leu Met Asp Ile Lys Ser Arg Leu Glu Gln
1 5 10 15

<210> 96
<211> 15
<212> PRT
<213> Artificial

<220>
<223> CYFRA protein

<400> 96

Met Lys Ala Ala Leu Glu Asp Thr Leu Ala Glu Thr Glu Ala Arg
1 5 10 15

## Description

The present invention relates to the assessment of lung cancer in a blood sample using a combination of biomarkers, namely CYFRA 21-1 and leucine-rich alpha-2 glycoprotein 1 (LRG-1). Measurement of the combination may be used for the early detection or for the follow-up of patients having lung cancer, including NSCLC cancer, with high sensitivity and selectivity.

### Background

Lung cancer is the most common cause of death from cancer and each year 1.4 million new cases are diagnosed worldwide. More than two-thirds of lung cancers are diagnosed at a late stage, when clinical symptoms appear. The overall survival rate after diagnosis ranges from 14 % in the USA to 1.1 % in some regions of Asia (Stewart and Kleihues, 2003) and is currently very low due to the late diagnosis of the disease. Current diagnostics methods are based on imaging techniques and invasive procedures such as bronchoscopy or biopsy(Rivera et al., 2003). The few known plasma biomarkers for lung cancer, such as carcinoembryionic antigen (CEA), squamous cell carcinoma antigen (SCC) and neuron-specific enolase (NSE) lack sufficient sensitivity and specificity (Kulpa et al., 2002) to be used as early diagnostics tools. Ongoing efforts using high throughput discovery technologies are still struggling to provide reliable and easily accessible lung cancer biomarkers to enter the clinic(Zolg, 2006; Beane et al., 2009). Stieber et al., 2006 is a review of the current state of knowledge of the clinical use of serum-based markers.

Global proteome analysis has been hampered by a variety of methodological problems including the fact that current mass spectrometry based proteome profiling technologies are in general far from covering the necessary dynamic range; are not adequately sensitive and lack sufficient reproducibility and throughput (Rifai et al., 2006). Polyclonal and monoclonal antibodies (mAbs) provide appropriately validated tools for the characterization and quantitative analysis of proteins but have primarily been targeted at a set of epitopes with low complexity (Gao, 2005; Knezevic, 2001). Global, antibody proteomics approaches aim to generate libraries of antibodies to cover most or all individual proteins and their immunogenic epitops in any complex proteome. Recombinant phage, bacterial and viral display represent approaches to global antibody generation but have had limited success in resulting of libraries capable to detect complex proteomes with sufficient quality affinity reagents and coverage (Maneewatch, 2009; Houimel and Dellagi, 2009). The Human Protein Atlas (Uhlen, 2005) and other large initiatives such as the NCI Clinical Proteomics Technology Initiative (Blow, 2007) are targeted at the generation of comprehensive libraries to the far more complex human proteome with both approaches using recombinant proteins as immunogens. The problem with recombinant proteins is that they do not represent the protein natural state, lack post-translational modifications and correct folding, therefore limiting the potential of the obtained libraries to profile natural proteomes.

### Summary

The present disclosure relates to the assessment or monitoring of lung cancer ("LC") using a combination of protein biomarkers. It discloses the use of a CYFRA protein in combination with either one of the following proteins or their combinations: haptoglobin (HPT), alpha-1-antichymotrypsin (ACT), leucine-rich alpha-2 glycoprotein 1 (LRG-1), and complement factor 9 (C9), for detecting or monitoring lung cancer in human subjects. Surprisingly, the application shows that measurement of anyone of these combinations allows the early detection or the follow-up of patients having lung cancer, including NSCLC cancer, with remarkably high sensitivity and selectivity.

The object of this invention relates to methods and uses as defined in the claims. More particularly, the invention relates to a method for the detection, diagnosis or staging of lung cancer in a-patient, the method comprising the combined analysis of CYFRA 21-1 and LRG-1 proteins, in a blood sample from the subject, said combined analysis providing an indication of the presence, stage or progression of lung cancer in the patient.

The invention is particularly suited to detect or monitor lung cancer in human subjects, particularly Stage I and II, e.g., Stage Ia and IIa lung cancer.

Another object of the invention relates to the use of CYFRA 21-1 in combination with LRG-1 for the diagnosis and/or management of LC. Another object of the invention relates to the use of CYFRA 21-1 in combination with at least LRG-1 for the diagnosis and/or management ofLC.

Also disclosed is a method for the detection, diagnosis, staging or management of LC in a patient, the method comprising the combined analysis of (i) a CYFRA protein, and (ii) at least a second protein selected from ACT, HPT and C9, in a sample from the subject, said combined analysis providing an indication of the presence, stage or progression of lung cancer in the patient.

The term "combined analysis" indicates that the cited markers are tested (e.g., for their presence, amount, relative expression level, variations, etc.) in the sample, either simultaneously or separately. It is the combined measure or variation of the at least two markers which provides a relevant and specific indication of the disease.

In a particular embodiment, the method comprises providing a blood sample from the subject, contacting said sample, or an aliquot thereof, with a first specific binding reagent that binds a CYFRA 21-1 protein and with at least a second specific reagent that binds a LRG-1 protein, and determining the presence or amount of protein bound to said binding reagents. Contacting with the at least 2 binding reagents can be performed simultaneously or sequentially, on (a same aliquot of) the same sample or on different aliquots of a same sample. The binding reagents are, most preferably, antibodies or fragments thereof retaining antigen specificity.

In another particular embodiment, the anti-LRG-1 antibody or a fragment thereof is an antibody or a fragment thereof which binds human LRG-1 and which binds a peptide having a sequence selected from SEQ ID NOs: 26-35 and 57-63.

In another particular embodiment, the anti-CYFRA 21-1 antibody or a fragment thereof is an antibody or a fragment thereof which binds human cytokeratin 19 fragment, CYFRA 21-1, or a protein or peptide comprising a sequence selected from SEQ ID NOs: 93-96.

Legend to the figures

Figure 1. A dot-plot showing CYFRA 21-1, ACT, LRG-1, C9 and Hpt as measured by ELISA in plasma samples from healthy controls (n=158) and lung cancer patients (n=230).

The statistics are shown as box plots to each group where the bottom and top of the box are the $25^{th}$ and $75^{th}$ percentiles; the band in the box is the median value.

Figure 2. Standard curves of sandwich ELISA assays with **(a)** anti-ACT antibodies; **(b)** anti-beta-HPT antibodies; **(c)**anti-CFH antibodies; **(d)** anti-LRG antibodies; **(e)** anti-C9 antibodies.

Figure 3. Receiver operator curves (ROC) analysis of the diagnostic performance of single biomarkers and combinations of biomarkers.

(a) **all stages of LC:** blue - *LRG,* AUC=0.85; black- *CYFRA,* AUC=0.91; red-*CYFRA* + *LRG,* AUC=0.93; green-*CYFRA* + *LRG* + *ACT* + *C9* + *Hpt* + *CFH,* AUC=0.94;
(b) **only stage I of LC** :blue - *LRG, AUC=0.88; black- CYFRA, AUC=0.92; red-CYFRA + LRG, AUC=0.95; green- CYFRA + LRG + ACT + C9 + Hpt + CFH, AUC=0.96;*
**(c) stages II, III and IV of LC:** blue *- LRG, AUC=0.92; black- CYFRA, AUC=0.91; red - CYFRA + LRG, AUC=0.96; green- CYFRA + LRG + ACT + C9 + Hpt + CFH, AUC=0.97;*

Detailed description of the invention

A challenge in the treatment of lung cancer is the lack of early, pre-symptomatic detection as lung cancer symptoms generally present at advanced stages. Here, we describe biomarker combinations which allow specific, reliable and sensitive detection and staging of lung cancer in human subjects.

International application No PCT/EP2010/061354, presently unpublished, discloses the discovery of cancer specific biomarkers. In the present application, specific combinations of some of these biomarkers with CYFRA were tested and shown to exhibit remarkably high levels of selectivity and specificity for diagnosing and managing lung cancer.

The inventors have measured the concentration of the different biomarkers in plasma of lung cancer patients and healthy subjects using sandwich ELISA assays. HPT, ACT, LRG-1, and C9 proteins were measured using SW ELISA assays (Fig.1), and a CYFRA protein (e.g., cytokeratin-19 fragment) was measured using a commercially available kit CYFRA 21-1 EIA (Fujirebio). The present disclosure shows the use of CYFRA, in combination with either one of the above 4 biomarkers or their combinations provides a reliable assessment of the presence, stage or progression of lung cancer in human subjects. Measurement of anyone of these combinations may be used for the early detection or for the follow-up of patients having lung cancer, including NSCLC cancer, with high sensitivity and selectivity. Furthermore, the above biomarker combinations may be used in further combination with additional markers, such as e.g., Complement factor H (CFH), depending on the disease stage. The method is particularly suited to detect stage I and II, more particularly stage Ia and IIa, lung cancer.

Combinations of each of these individual biomarkers with CYFRA, as well as multimember panels of all biomarkers, were tested and showed remarkable diagnostic performance (measured as area under the curve of the ROC), higher than 0.9, and sensitivity up to 84 % at 95 % specificity.

Altogether, the disclosure thus provides novel combinatorial biomarkers for clinical applications in lung cancer patient detection, management, diagnosis and monitoring.

Disclosed is a method of detecting, diagnosing, staging or managing lung cancer in a subject, based on a combined analysis of at least two markers, a CYFRA protein and at least one second protein selected from LRG-1, ACT, HPT, and C9.

The invention in its broadest sense is a method as defined in independent claim 1 and a use as defined in independent claim 9.

Within the context of the invention, the use of a protein for cancer detection, diagnosis or management includes, without limitation, the use of the protein (in any form, soluble or not, full length or not), or of any coding nucleic acids, as a biomarker. This includes, e.g., the use of any reagent to detect or quantify (i) the protein or any variant or mutant thereof, such as splicing variants or polymorphisms, and/or (ii) any nucleic acid encoding said proteins, such as DNA or RNA, said protein and/or nucleic acid levels being correlated to the disease. The term also includes any measure of the expression level of the cited protein, and a comparison of the measured level to a reference or mean value. The measured amount or level or information provides an indication regarding the specified disease in the subject.

CYFRA

The term CYFRA designates any Cytokeratin protein or any fragment of a cytokeratin protein. More preferably, the term CYFRA designates any soluble or circulating form of a cytokeratin protein or fragment. The invention is limited to CYFRA 21-1 as the CYFRA.

Three major cytokeratins are found in simple epithelia: Cytokeratin 19 (GI: 127799941, illustrative sequence is shown in SEQ ID NO: 87 below), the smallest human keratin; Cytokeratin 8, and Cytokeratin 18. Cytokeratin 19 is a major keratin in carcinomas (Chu et al., 2002). During apoptosis, soluble cytokeratin fragments are produced from epithelial cells by caspase-mediated proteolysis (Dohmoto et al., 2001). One of these fragments is a fragment of Cytokeratin 19, designated CYFRA21-1, which is produced by cleavage at 234SVEVD caspase-cleavable sequence, resulting in a 18.4 kDa fragment that corresponds to amino acid residues 234 to 400 of the sequence of SEQ ID NO: 87. CYFRA21-1 was proposed as a biomarker for lung cancer (Stieber et al., 1993; Pujol et al., 1993 ; or Ebert et al., 1994). Recent evidence further show that tumour cells may also release full-length cytokeratin 19 (Alix-Panabieres et al., 2009). However, the CYFRA21-1 fragment or full length cytokeratins, alone, do not represent reliable biomarkers sufficient to provide a specific and sensitive LC diagnostic assay.

As indicated above, the inventors have now surprisingly discovered that the use of CYFRA, in combination with either one of C9, HPT, LRG-1, ACT or CFH, or their combinations, provides a reliable assessment of the presence, stage or progression of lung cancer in human subjects. Measurement of anyone of these combinations may be used in particular for the early detection or for the follow-up of patients having lung cancer, including NSCLC cancer, with remarkable diagnostic performance substantially higher than each marker individually, as illustrated e.g., by a sensitivity as high as 84 % at 95 % specificity.

Detecting a CYFRA protein, or using CYFRA as disclosed, designates the detection of any form of CYFRA (e.g., full length or fragments thereof, preferably soluble forms thereof) or corresponding nucleic acids. Specific examples of a CYFRA protein include full length Cytokeratin 19, CYFRA21-1 fragment, and any polypeptide comprising a sequence selected from SEQ ID NO: 93 to 96.

The presence or amount of a CYFRA protein can be detected using a binding reagent, particularly a specific antibody (or fragments or derivatives thereof retaining antigen specificity). In a preferred embodiment, the invention uses an anti-CYFRA21-1 antibody which binds a human cytokeratin 19 epitope. More preferably, the invention uses an antibody that binds an epitope contained in the amino acid of human cytokeratin 19 represented below.

More specific examples are antibodies which bind a peptide selected from SEQ ID NO: 93-96 and which also binds a human cytokeratin protein, or a fragment or derivative of such an antibody having the same antigen specificity. Specific examples of such antibodies include monoclonal antibodies BM19.21 and KS 19.1.

Cytokeratin 19 fragments (e.g., CYFRA21-1) was measured using a commercially available assay based on monoclonal antibodies The epitope mapping of human keratin 19 of the specificities of antibodies BM 19.21 (Böttger V. et al., Eur J Biochem 1995;231(2):475-85) and KS 19.1 (Achtstatter, T. (1988) Koexpressionsmuster von Intermediiirfilament-Proteinen, University of Heidelberg) using synthetic peptides is shown below:

| SEQ ID | Sequence | Ab |
|---|---|---|
| 93 | DVRADSERQNQEYQR | BM 19.21 |
| 94 | SERQNQEYQRLMDIK | |
| 95 | QEYQRLMDIKSRLEQ | |

(continued)

| SEQ ID | Sequence | Ab |
|---|---|---|
| 96 | MKAALEDTLAETEAR | KS 19.1 |

The mapping of the synthetic peptides recognized by the two antibodies on the sequence of human keratin 19 is shown on the following sequence. BM 19.21 recognizes an epitope localized between residues 351 and 375 and KS 19.1 recognizes an epitope localized between residues 316 and 330.

Amino Acid sequence of Human Cytokeratin 19 (SEQ ID NO: 87):

```
  1 MTSYSYRQSS ATSSFGGLGG GSVRFGPGVA FRAPSIHGGS GGRGVSVSSA RFVSSSSSGG
 61 YGGGYGGVLT ASDGLLAGNE KLTMQNLNDR LASYLDKVRA LEAANGELEV KIRDWYQKQG
121 PGPSRDYSHY YTTIQDLRDK ILGATIENSR IVLQIDNARL AADDFRTKFE TEQALRMSVE
181 ADINGLRRVL DELTLARTDL EMQIEGLKEE LAYLKKNHEE EISTLRGQVG GQVSVEVDSA
241 PGTDLAKILS DMRSQYEVMA EQNRKDAEAW FTSRTEELNR EVAGHTEQLQ MSRSEVTDLR
301 RTLQGLEIEL QSQLSMKAAL EDTLAETEAR FGAQLAHIQA LISGIEAQLG DVRADSERQN
361 QEYQRLMDIK SRLEQEIATY RSLLEGQEDH YNNLSASKVL
```

Other antibodies may be found or generated against a CYFRA protein and used in the present invention. It should be noted, however, that the use of antibodies that bind an epitope present in any one of SEQ ID NOs: 93-96 is particularly preferred.

<u>CYFRA / LRG-1 combinations</u>

The leucine-rich repeat (LRR) family of proteins, including LRG-1, has been shown to be involved in protein-protein interaction, signal transduction, and cell adhesion and development. LRG1 is expressed during granulocyte differentiation (O'Donnell et al., 2002). Human LRG1 was isolated from human serum by Haupt and Baudner, 1977. By sequence analysis, Takahashi et al. (1985) determined that purified LRG1 protein has 312 amino acids and an experimentally determined molecular mass of 45 kD. The LRG1 polypeptide contains 1 galactosamine and 4 glucosamine oligosaccharides attached and has 2 intrachain disulfide bonds. Leucine comprises 66 of the 312 amino acids, and LRG1 contains at least 8 24-amino acid leucine-rich repeats. Increased LRG1 expression was detected in GCSF-treated human cells derived from a patient with myeloproliferative disorder. In contrast, decreased LRG1 expression was detected after PMA treatment and induction of monocytic differentiation of HL-60 cells

A number of proteomics studies focused on plasma and serum have demonstrated an association between elevated levels of leucine rich alpha-2 glycoprotein (LRG1) and the presence of a number of different cancers in multiple preliminary studies. However, none of the findings have been confirmed by clinical validation. All of these studies are based on proteomics and none involved the use of antibodies.

The invention discloses that LRG1, in combination with CYFRA, represents highly predictive lung cancer-specific biomarker combination. In particular, the results obtained demonstrate that a combined analysis of CYFRA and LRG-1 substantially increases the reliability and sensitivity of lung cancer detection. In particular, the combined assessment of CYFRA and LRG-1 allows the detection of lung cancer with a sensitivity of 83.25% at 95% specificity while, individually, CYFRA and LRG-1 provide a sensitivity of 67.39 and 62.26, respectively.

In this respect, the invention discloses the use of CYFRA in combination with LRG-1 for the detection, diagnosis and/or management of LC.

The invention relates to the use of CYFRA in combination with LRG-1 and in further combination with at least a protein selected from ACT, HPT, and C9, for the detection, diagnosis and/or management of LC. The results show that sensitivity of detection may be further increased by adding a limited number selected biomarkers. In particular, the combined assessment of CYFRA, LRG-1, C9 and ACT allows the detection of lung cancer with a sensitivity of 84.24% at 95% specificity. Such a combined assessment represents a preferred embodiment of the invention.

The invention also discloses a method for the detection, diagnosis, staging or management of LC in a patient, the method comprising the combined analysis of a CYFRA protein and an LRG-1 protein, in a blood sample from the subject, said combined analysis providing an indication of the presence, stage or progression of lung cancer in the patient. In a further preferred embodiment, the method comprises the analysis of at least one further protein selected from ACT, HPT, C9 and CFH.

The invention also discloses a method for detecting lung cancer in a subject, the method comprising contacting a sample from said subject, which is a blood sample, with a first antibody, fragment or derivative thereof, that binds a CYFRA protein and with at least a second antibody, fragment or derivative thereof, that binds LRG-1, and determining the presence of a binding, said presence being indicative of lung cancer.

Also disclosed is a device comprising at least one binding reagent (e.g., an antibody, fragment or derivative thereof) that binds a CYFRA protein and at least one binding reagent (e.g., an antibody, fragment or derivative thereof) that binds a LRG-1 protein, immobilized on a support. For example, the support is a membrane, a slide, a microarray, a chip or a microbead.

Any anti-LRG-1 antibody may be used to carry out the present invention. However, in a preferred embodiment, the invention uses an anti-LRG-1 antibody which binds a peptide selected from SEQ ID NO: 26-35 and 57-63 and which also binds a human LRG1 (Leucine Rich Alpha -2 Glycoprotein 1) protein, or a fragment or derivative of such an antibody having the same antigen specificity. The invention particularly uses an antibody which binds a peptide selected from SEQ ID NO: 26-35 and 57-63 and wherein said binding is at least partially displaced by a human LRG-1 protein, or a fragment or derivative of such an antibody having the same antigen specificity. Specific examples of such antibodies include Bsi0392; Bsi0351 and Bsi0352, as disclosed in the experimental section. A further example includes monoclonal antibody 2F5.A2 produced by hybridoma having ATCC accession number PTA-8131 (US Patent No. US7,416,850). This patent also discloses a specific assay method for detecting LRG-1, using Cytochrome C and antibody 2F5.A2, which may be specifically used for carrying out the present invention.

<u>CYFRA, LRG-1, C9 and ACT combination</u>

The invention discloses the combined determination of CYFRA, LRG-1, C9 and ACT. Indeed, the results show that the sensitivity of detection may be optimal using such a combination CYFRA, LRG-1, C9 and ACT, especially for detection of stage I LC. The invention thus discloses a method of detection, diagnosing, staging or monitoring LC in a human subject, the method comprising the combined determination of CYFRA, LRG-1, C9 and ACT in a blood sample from the subject, said determination being indicative of LC. The invention also discloses a device comprising reagents for combined detection of CYFRA, LRG-1, C9 and ACT. Furthermore, although very predictive in itself, this marker combination of the invention may be further combined with other biomarker(s).

<u>Assay Format and Implementation</u>

The invention may be performed using different types of blood samples, various types of antibodies or binding reagents, and different assay formats.

The sample is blood, serum or plasma, particularly whole blood. The sample may be treated prior to being used in the method of the invention, e.g., for dilution, enrichment, concentration, filtration, etc. Also, various aliquots of a sample may be prepared, for separate testing.

The invention preferably utilises binding reagents that specifically or selectively bind to a biomarker. Selective or specific binding indicates that binding to another molecule can be discriminated from (e.g., occurs with higher affinity or avidity than) specific binding to the target biomarker. Preferred reagents do not bind, under selective condition, to any other unrelated human blood protein but the reference protein. Binding of a reagent to a reference molecule can be tested as disclosed in the examples.

The binding reagents may be selected from aptamers, specific ligands, antibodies, or derivatives thereof.

In a particular embodiment, the binding reagent is an antibody. The antibody may be a polyclonal or a monoclonal antibody, most preferably a monoclonal. It may be of various classes (e.g., IgG, IgE, IgM, etc.). The antibody may be of various animal origin, or human or synthetic or recombinant. Furthermore, the term antibody also includes fragments and derivatives thereof, in particular fragments and derivatives of said monoclonal or polyclonal antibodies having substantially the same antigenic specificity. Antibody fragments include e.g., Fab, Fab'2, CDRs, etc. Derivatives include humanized antibodies, human antibodies, chimeric antibodies, poly-functional antibodies, Single Chain antibodies (ScFv), etc. These may be produced according to conventional methods, including immunization of an animal and collection of serum (polyclonal) or spleen cells (to produce hybridomas by fusion with appropriate cell lines).

Methods of producing polyclonal antibodies from various species, including mice, rodents, primates, horses, pigs, rabbits, poultry, etc. may are selected from techniques known per se in the art. Briefly, the antigen is combined with an adjuvant (e.g., Freud's adjuvant) and administered to an animal, typically by sub-cutaneous injection. Repeated injections may be performed. Blood samples are collected and immunoglobulins or serum are separated.

Methods of producing monoclonal antibodies from various species as listed above may be found, for instance, in Harlow et al., 1988 or in Kohler et al., 1975. Briefly, these methods comprise immunizing an animal with the antigen, followed by a recovery of spleen cells which are then fused with immortalized cells, such as myeloma cells. The resulting hybridomas produce the monoclonal antibodies and can be selected by limit dilutions to isolate individual clones. Antibodies may also be produced by selection of combinatorial libraries of immunoglobulins, as disclosed for instance in Ward et al., 1989.

Recombinant antibodies, or fragments or derivatives thereof, may be produced by methods known per se in the art, for example by recombination in a host cell, transformed with one or more vectors enabling the expression and/or secretion of the nucleotide sequences encoding the heavy chain or the light chain of the antibody. The vector generally contains a promoter, translation initiation and termination signals, and suitable transcriptional regulatory regions. It is stably maintained in the host cell and may optionally possess specific signals for secretion of the translated protein. These different components are selected and optimized by one of skill in the art according to the host cell used.

For use in the invention, the antibodies may be coupled to heterologous moieties, such as labels, tags, linkers, etc.

The method of the invention may be carried out using a variety of detection technologies or platforms known per se in the art such as, without limitation Capture assay, Sandwich assay, Competition assay, Radio-immuno assays, Enzyme labels with substrates that generate colored, fluorescent, chemiluminescent, or electrochemically-active products, Fluorescence, fluorescent polarization, Chemiluminescence, Optical and colorimetric, Electrochemiluminescence, Time-resolved fluorescence, Surface plasmon resonance, Evanescent wave, Multiwell plate (ELISA), Individual assay, Multiplex assay, Latex bead - multiplex assay, Microarray (Laminar surface) - multiplex assay, Glass, Ceramic (like Randox), Plate based assays, Strip based assays, dipsticks, Closed systems immunoassays. Preferred assay formats include:

*Capture assay*

An assay carried out using a single immobilized antibody (multiwall plate, latex bead, microarray, etc.) which captures a specific labeled protein from a biofluid, the detection which is measured using appropriate detection reagents as detailed in the following paragraph. The antibody is immobilized directly to the support or captured by an affinity reagent such as an anti-mouse IgG antibody coated onto the support. The immobilized antibody is then incubated with any of the above mentioned body fluids in which the proteins have been labeled with a detection molecule such as biotin, with or without pre-treatment to remove abundant proteins. The labeled protein which is bound by the antibody is detected by the addition of an appropriate detection reagent which binds to the label such as avidin or streptavidin which has been modified to be compatible with one of the detection technologies described in the section "detection technology." The resulting signal provides a quantitative measure of the amount of labeled protein bound by the antibody

*Sandwich ELISA*

An assay using two antibodies, the first which is immobilized on a support (multiwell plate, latex bead, microarray, etc.) which binds a specific protein from a biofluid, the detection which is measured using a labeled second antibody against the same protein and appropriate detection reagents as detailed in the following paragraph.

The first antibody is immobilized directly to the support or captured by an affinity reagent such as an anti-mouse IgG antibody coated onto the support. The immobilized antibody is then incubated with any of the above mentioned body fluids, with or without pre-treatment to remove abundant proteins. The antibody/antigen complex is then incubated with a second antibody, made against the same protein, which has been labeled with a detection molecule such as biotin. The bound antibody is detected by the addition of an appropriate detection reagent which binds to the label such as avidin or streptavidin which has been modified to be compatible with one of the detection technologies described in the section "detection technology." The resulting signal provides a quantitative measure of the amount of protein bound by the antibody

*Competitive assay*

An assay in which the binding of a labeled tracer protein by a single antibody as described in "capture assay" is inhibited by pre-incubation of a biofluid to indirectly quantify the analyte. The antibody is immobilized directly to the support or captured by an affinity reagent such as an anti-mouse IgG antibody coated onto the support. The immobilized antibody is then incubated with any of the above mentioned body fluids. The immobilized antibody/antigen complex is then incubated with a labeled tracer consisting of either (1) any of the above mentioned body fluids in which the proteins have been labeled with a detection molecule such as biotin, with or without pre-treatment to remove abundant proteins, or (2) a purified or recombinant protein recognized (bound) by the monoclonal antibody, or (3) a peptide which is recognized (bound) by the monoclonal antibody. The labeled protein or peptide which is bound by the antibody is detected by the addition of an appropriate detection reagent which binds to the label such as avidin or streptavidin which has been modified to be compatible with one of the detection technologies described in the section "detection technology." The level of the specific protein in the unlabeled biofluid is determined as a function of the inhibition of signal.

Preferred Detection Technologies include:

Enzyme labels with substrates that generate colored, fluorescent, chemiluminescent, or electrochemically-active products.

The detection reagent (for example steptavidin or avidin, which binds to biotin) is coupled to an enzyme such as horseradish peroxidase which is capable of catalyzing

an appropriate colorimetric substrate of which the product demonstrates maximal absorbance at a given wavelength allowing the quantitative measurement of the labeled protein by measuring the optical density of the final product in the well at or near the wavelength of maximal absorbance.

a chemiluminescent substrate to a sensitized reagent which upon oxidation emits light, providing the quantitative measurement of the labeled protein.

a chemiluminescent substrate to a sensitized reagent which upon the application of an electrical current emits light, providing the quantitative measurement of the labeled protein Fluorescence

The detection reagent (for example steptavidin or avidin, which binds to biotin) is coupled to a fluorescent tag.

Preferred platform Technologies include:

Multiwell Plate

Single test: one antibody is immobilized per well either directly or indirectly using a capture reagent such as goat anti-mouse antibody.

Multiplex: 2 or more antibodies are immobilized in a single well by deposition in a pattern

Latex bead

Two or more antibodies are immobilized onto a latex bead between x and y microns

Arrays, microarrays, and nanoarrays

Two or more antibodies are spotted onto an activated laminar surface with a spot diameter between 100 μm - 5 mm (arrays), 2 μm - 100 μm (microarrays), 10 nm-2 μm (nano-arrays) The surface can be composed of glass, plastic, ceramic, carbon nanotube lattice etc.

Different methods or algorithms can be used to derive the lung cancer index. In the simplest form, the concentrations of the individual markers measured in the sample are combined using a linear function (see below) to derive an index value which indicates the presence of lung cancer if the index value is above a pre-determined threshold.

$$\text{Index} = A^* \text{ Concentration (BM1)} + B^* \text{ Concentration (BM2)} + C^* \text{ Concentration(BM3)} \ldots$$

Where BM designates a biomarker.

Another method or algorithm consists of first proceeding to the standardization of the obtained individual concentration measurements using pre-determined values for the mean and the standard deviation (std) (see below) for each biomarker as to bring the measurements of biomarkers in different concentration ranges to the same scale, as follows:

$$\text{Standardized Value (BM1)} = ((\text{Concentration(BM1)} - \text{Mean(BM1)})/\text{Std(BM1)})$$

The standardized values are then used as described in the previous paragraph. The standardization procedure can also be used to correct for reproducible changes in the level of the biomarker concentrations that can be attributed to analytical factors.

The method of the invention can be performed at any stage of the disease, such as early or late stage, to confirm or reject a prior diagnosis, select patients for surgery, classify cancer type or severity, or monitor patients. The test may also be conducted before disease symptoms, as a first line detection. Clinical diagnostic application of a lung cancer plasma (serum) test will be useful e.g., for patients who are suspected to have lung cancer because of a suspicious nodule that has been detected by imaging of the lung (CT scan). Nodules < 0.5 cm are suspicious and quite frequent in the populations. Nodules > 0.5 cm, but < 1.1 cm represent an increased likelihood of being cancerous, however challenging to find by surgery and invasive endoscopic procedures. It is important to select patients from this group with nodules that are definitively cancerous to reduce the burden of futile surgeries and other invasive diagnostic procedures.

The test will avoid futile thoracotomies and unnecessary and expensive imaging technologies that are not specific enough and expose the patients to potentially harmful irradiation, and missed cures as observed patients could receive the test repeatedly.

The invention is particularly advantageous as it allows detection of Stage I or II, in particular Stages Ia and stage IIa LC in human subjects.

Further aspects and advantages of the invention will be disclosed in the following examples, which should be considered illustrative. Only examples falling under the claims constitute the invention.

Examples

**A. Methods**

Clinical samples

Plasma samples from patients with newly diagnosed lung cancer and no previous treatment were obtained from Asterand (Royston, UK) and from the Department of Pulmonology of the University of Debrecen in Hungary from informed patients and apparently healthy individuals after obtaining their written consent by a clinical protocol RKEB/IKEB:2422-2005 approved by the regional ethics committee and the IRB of the clinic (Table 1). Lung cancer staging was done according to the American Joint Committee on Cancer (AJCC) and was based on information in the final histopathology report having the LC-histotype according to the WHO classification (19). Clinical data including stage at diagnosis, histology, additional pulmonary pathologies, smoking habits and general patient demographics are presented in Table1.

| Cohort | Status | Number subjects | Age | Gender* | Smoking status | packs/day (avg) | Stage LC | Histology† | Other LD‡ |
|---|---|---|---|---|---|---|---|---|---|
| Training set (Asterand) | LC | 219 | 26-86 (avg 61) | M: 158<br>F: 61 | Current Use: 116<br>Occasional use: 5<br>Previous use: 63<br>Never used: 32<br>unknown: 3 | 1.3 | I: 128<br>II: 39<br>III: 31<br>IV: 5<br>unknown: 16 | SQC: 104<br>AC: 85<br>Other NSCLC: 25<br>SCLC: 5 | COPD: 6<br>Emphysema: 5<br>Chronic bronchitis: 21 Asthma: 3 |
| | Healthy | 169 | 18-70 (avg 38) | M:114<br>F: 55 | Current Use: 49<br>Occasional use: 4<br>Previous use: 25<br>Never used: 91 | 1.0 | - | - | - |
| Test set Univ. Of Debrecen | LC | 45 | 44-77 (avg 59) | M: 32<br>F: 13 | Current Use: 45 | 1.7 | I: 1<br>III: 1<br>III-IV: 12<br>IV: 18<br>unknown: 13 | SQC: 14<br>AC: 10<br>Other NSCLC: 4<br>SCLC: 17 | COPD: 22 |
| Healthy | 63 | 34-72 (avg 54) | M: 23<br>F: 40 | Current Use: 63 | 1.1 | - | - | | COPD: 0 |

Antibody purification

Ascites fluid was produced by intraperitoneally injecting 1-5x106 cloned hybridoma cells into incomplete Freund's adjuvant-primed Balb/c mice, using a protocol which was approved by the animal care committee of the animal facility. Ascitic fluid was cleared of cells and cell debris, and IgG was purified using a two-step affinity chromatography procedure consisting of pre-purification on a Pierce thiophilic adsorbent column from Thermo Fisher Scientific (Waltham, MA) followed by further purification using Protein-G Sepharose 4 FF from GE Healthcare (Chalfont St. Giles, UK) . In both cases the procedures were carried out as recommended by the suppliers. The purified antibodies were concentrated by ultrafiltration. The purities of the preparations were assessed by SDS-PAGE, and the protein concentration was determined using the BCA protein assay kit from Pierce (Rockford, IL). The purified antibodies were stored as working aliquots at -78 oC.

Biotinylation of mAbs

Purified monoclonal antibodies were labeled with a bifunctional NHS-biotin having a long alkyl chain as a spacer EZ-Link Sulfo-NHS-LC-Biotin from Pierce (Rockford, IL). Labeling was performed in PBS buffer (pH 7.0) at a 50 time molar excess of biotin for 30 min. at room temperature.

Biomarker quantification

The different biomarkers were quantified with sandwich ELISA assays using the Abs couples listed in the Table below. The 384-well microtiter plates (high binding plates from Corning) were directly coated with the capture mAbs at 20 μg/ml and then blocked with PBS buffer containing 0.5% BSA (w/v). The plasmas and the protein calibrators, diluted in PBS containing 0.05% (v/v) Tween 20 and 1% FBS (v/v), were then incubated in the wells. The binding of the biotinylated detection antibodies, added at 5 μg/ml and washed 4 times with PBS containing 0.05% (v/v) Tween 20 was then followed by using an avidin complex coupled to the HRP (VECTASTAIN® ABC kits) and its substrate (TMB). The reaction was measured using kinetic readings at 650nm during 2min at 37°C.

LRG has been also quantified with a second type of assay based on the affinity of the LRG for the Cytochrome C using the protocol described in Weivoda et al. (J Immunol Methods. 2008 Jul 20;336(1):22-9. Epub 2008 Apr 4).

The standard curves obtained with the calibrators have been plotted using the four-parameter fitting algorithm. The concentrations of the analytes in the plasmas were calculated from the corresponding standard curves.

CYFRA21-1 was measured using a commercially available assay based on monoclonal antibodies BM 19.21 and KS 19.1 (Cyfra 21-1 EIA kit from Fujirebio Diagnostics, Inc. (ref. 211-10).

The monoclonal antibodies and reagents used in this work are listed below:

| Capture reagent | Detection Ab | Target Protein |
|---|---|---|
| BM 19.21 | KS 19.1 | CYFRA21-1 |
| Bsi0272 | Bsi0452 | C9 |
| Bsi0352 | 2F5.A2* | LRG-1 |
| Cytochrome c | Bsi0392 | LRG-1 |
| Bsi1709 | Bsi0033 | Haptoglobin |
| Bsi0186 | Bsi0358 | ACT |
| * Weivoda S, et al., J Immunol Methods. 2008 Jul 20;336(1):22-9 | | |

Immunisation of BALB/c mice with complex immunogens

Female Balb/c mice of at least 8 weeks of age from Charles River Laboratories (Evry, France) were injected subcutaneously in the rear footpads and at the base of the tail with the two complex antigen protein mixtures. Each mice received 10 μg protein of complex immunogen preparation on days 1, 15 and 29. Complete Freund's adjuvant (Sigma, Saint Louis, MO) was used in all cases. Blood was taken from each mouse by retro-orbital bleed using a Pasteur pipette on days 19 and 33 to monitor antibody production by ELISA. Three weeks minimum after the third immunization (day 52 for group A and day 61 for group B) an additional injection with 10 μg of the complex antigen mixture in PBS pH 7.0 was performed to boost the immune response. Mice were treated in an animal facility according to the French and European laws and regulations regarding animal experimentation.

Fusion, cell growth and cloning

The procedures were done according to published methods. In short; Sp2/0-Ag-14 cells were fused to splenocytes of immunized mice with the help of polyethylene glycol (PEG). Fused cells were seeded to provide quasi clonal distribution of hybrids, which were selected in HAT media. Hybridoma cell lines were cloned before antibody production (in-vitro or in-vivo)

High throughput direct-ELISA Screening

All chemicals, unless specified, were obtained from Sigma (St. Louis, MO). 384 well high-binding plates (Corning Inc., Lowell, MA) were coated with 20 μg/ml (13 μl per well) goat anti-mouse Ig gamma chain specific polyclonal antibody (GAM) from Southern Biotechnology Associates, Inc. (Birmingham, AL) in coating buffer at pH 9.6 for 2h at room temperature (RT). The plates were washed four times with 80 μl/well of PBS containing 0.05% (v/v) Tween (washing buffer) and blocked with 40 μl of PBS and 0.5% bovine serum albumine (BSA) at 4 °C overnight. Supernatants from the nascent hybridomas or from the clonal cell lines were added non-diluted to the wells. Each hybridoma supernatant was added to four adjacent wells to provide four independent readings. Mouse anti-human mAb against albumin was used as positive control and spotted in eight wells at 1.2 μg/ml in CM (13 μl/well). CM was used as negative control and also added to eight wells. The plates were incubated and then washed four times with washing buffer. All wells were incubated with biotinylated depleted plasmas (tracers) at 10 μg/ml in PBS with 0.05% (v/v) Tween and 1 % low IgG FBS. Incubation was continued for 90 min. at RT and the unbound proteins were removed by washing the plates four times with washing buffer. HRP-coupled avidin (Vectastain Elite ABC Peroxidase kit from Reactolab SA, Switzerland) was used as specified by the vendor's protocol. After four fold washing with washing buffer, reaction development was carried out by adding 20 μl freshly prepared substrate solution to each well (o-phenylenediamine at 0.4 mg/ml in 0.05 M phosphate/citrate buffer pH 5.0). The kinetics of the reaction development at 37 °C was followed at 450 nm by recording the absorbance multiple times. Liquid handling was performed using Multidrop Combi from Thermo (Waltham, MA), Multimek with 96 pin head from Beckman Coulter (Fullerton, CA) and STAR from Hamilton (Reno, NV). Plate washing was performed using ELX405 from BioTek (Winooski, VT). Absorbance was measured with a microplate reader SpectraMax from Molecular Device (MDS, Toronto, Canada).

Screening data analysis.

Vmax of the chromogenic reactions were calculated from the linear part of the kinetic readings using the software provided with the plate reader SoftMax Pro from Molecular Device (MDS, Toronto, Canada). Each plate had eight positive and negative controls used to calculate Z' factor, a metrics used to quantify the quality of the screening experiment with respect to reproducibility and data scatter [19]. Plates with a Z' factor below 0.5 (usually less than 10% in a screening campaign) were repeated. The positive (PC) and negative controls (NC) were used to normalize the data across plates and according to the following formula:

$$VmaxN_{sample} = (Vmax_{sample} - Vmax_{NC}) / (Vmax_{PC} - Vmax_{NC}).$$

Aberrant data (outliers) for each group of replicates (four per hybridoma sample reacted with one tracer and eight per control reacted with one tracer) were removed using automated procedure based on the mean and standard deviation values of the multiple measurements.

For each sample (*i*), the coefficient of variation (*CV*) is calculated on the *n* replicates. *CV* is a normalized measure of dispersion of the probability distribution and it is defined as the ratio of the standard deviation $\sigma$ to the mean $\mu$ as follows:

$$CV(i) = \sigma(i) / \mu(i)$$

If CV > 0.05, then a maximum ($T_{max}$) and a minimum ($T_{min}$) thresholds are calculated as follows:

$$T_{max}(i) = \mu(i) + 1.3 * \sigma(i)$$

$$T_{min}\ (i) = \mu\ (i) - 1.3 * \sigma\ (i)$$

The replicates which VmaxN is lower than $T_{min}$ or higher than $T_{max}$ are removed. In total 9.2 % of all generated data was considered as aberrant which represents the removal of less than one measurement for every two samples. The data obtained after normalizing and averaging the replicates (Figure 6) were further analyzed using statistical methods.

Statistical methods

The normality of the distribution of the results was estimated using the Wilks-Shapiro test. The distribution of the results for each hybridoma was calculated separately for the control and the lung cancer samples. Nonparametric statistical analyses were applied: differences between two independent groups were determined using the Mann-Whitney U test; differences between more than two groups were assessed using the Kruskal-Wallis one-way analysis of variance test. All statistical tests were two-sided and were performed using R statistical software (www.cran.r-project.org). A predictive model for discriminating lung cancer cases from healthy controls using the panel of five mAbs, was produced using the freely-available machine learning toolkit Weka (http://www.cs.waikato.ac.nz/~ml/) with a linear support vector machine and sequential minimal optimization algorithm (SMO). The model was established on the entire dataset using 10 fold cross validation.

Logistic regression based on the SMO predictions was calculated in order to produce probabilities of class values (here lung cancer vs. control) and to generate the Receiver Operating Characteristics (ROC) curves.

**B - Anti-LRG-1 antibodies**

Bsi0392

Bsi0392 is an IgG type monoclonal antibody. The heavy chain variable region amino acid sequence is represented in SEQ ID NO: 92, which is reproduced below (CDRs are underlined):

QIQLVQSGPELKKPGETVEISCKASGYTFTDYSMHWVKQAPGKGLKWMGWINTETGEPTYADDFKGRFAFSLETSATTAYLQINNLKNEDTATYFCARGGYYGNYDYAMDYWGQGTSLTVSS

Bsi0352

Bsi0352 is an IgG type monoclonal antibody. The heavy chain variable amino acid sequence is represented in SEQ ID NO: 89, which is reproduced below (CDRs are underlined):

EVQLQESGPSLVKPSQTLSLTCSVTGDSITSGSWNWIREFPGNKLEYMGYISYSGSTDYSPSLKSRISITRDTSKNQYYLQLNSVTTEDTATYYCATHYYGYLSLDYWGQGTSVTVSS

The difference in biomarker level with Bsi0352 is represented Fig 1, showing a very substantial difference between control and lung cancer. Peptides bound by Bsi0352 have been identified and verified. These peptides are presented as SEQ ID NOs: 26-35.

Bsi0351

Bsi0351 is an IgG type monoclonal antibody. The heavy chain variable amino acid sequence is represented in SEQ ID NO: 88, reproduced below (CDRs underlined).

DVQLQESGPGLVKPSQSLSLTCTVTGYSIINDYAWNWIRQFPGNKLEWMAYISYGGSIGYKPSLKSRISITRDTSKNQFFLQLNSVTTEDTATYYCARGGFYALDYWGQGTS

Peptides bound by Bsi0351 have been identified. These peptides are presented as SEQ ID NOs: 57-63.

**C - Anti-ACT antibodies**

Bsi0358

Bsi0358 is an IgG type monoclonal antibody. The heavy chain variable amino acid sequence is represented in SEQ ID NO: 90 reproduced below (CDRs underlined).

EVQLVESGGGLVQPKGSLKLSCAASGFTFNTYAMSWVRQAPGKGLEWISRIRSKS
NNYATYYVDSVKDRFTIYRDDSQSMLYLQMNNLKTEDTAIYYCVREGDWGQGTL
VTVSA

The difference in biomarker level with Bsi0358 is represented Fig 1, showing a very substantial difference between control and lung cancer. Peptides bound by Bsi0358 have been identified and verified. These peptides are presented as SEQ ID NOs: 1-13.

Bsi0359

Bsi0359 is an IgG type monoclonal antibody. The heavy chain variable amino acid sequence is represented in SEQ ID NO: 91 reproduced below (CDRs underlined).

EVQLVESGGGLVQPKGSLKLSCAASGFTFSTSAMNWVRQAPGKGLEWISRIRSKTN
NYATYYVDSVKDRFTIYRDDSQNMLYLQMNNLKTEDTAMYYCVREGDWGQGTL
VTVSA

Peptides bound by Bsi0359 have been identified and verified. These peptides are presented as SEQ ID NOs: 39-42.

**D - Anti-C9 antibodies**

Bsi0272

Bsi0272 is an IgG type monoclonal antibody. The heavy chain variable amino acid sequence is represented in SEQ ID NO: 86 reproduced below (CDRs underlined).

QVQLQQPGAELVRPGASVKLSCKASGYSFASYWMNWVKQRPGQGLEWIGMIHPS
DSGTSLDEKFKDKATLTVDKSSNTAYIQLNSPTSEDSAVYYCAREGYD.PAWFAY
WGQGTLVTVSA

The difference in biomarker level with Bsi0272 is represented Fig 1, showing a very substantial difference between control and lung cancer.

Peptides bound by Bsi0272 have been identified and verified. These peptides are presented as SEQ ID NOs: 14-25.

**E - Anti-HPT antibodies**

Bsi0033

Bsi0033 is an IgG type monoclonal antibody. The heavy chain variable amino acid sequence is represented in SEQ ID NO: 77 reproduced below (CDRs underlined).

EVQLQQSGADLVKPGASVKLSCTASGFNIKDTYMHWMKQRPEQGLEWIGRIDPAN
GNSKYDPKFQGKATITADTSSNTAYLQLSSLTSEDTAVYFCTKSAGVPFAYWGQG
TLVTVSA

The difference in biomarker level with Bsi0033 is represented Fig 1, showing a very substantial difference between control and lung cancer. Peptides bound by Bsi0033 have also been identified and verified. These peptides are presented as SEQ ID NOs: 36-38.

Bsi0071

Bsi0071 is an IgG type monoclonal antibody. The heavy chain variable amino acid sequence is represented in SEQ ID NO: 81 reproduced below (CDRs underlined).

EVMLVESGGGLVKPGGSLKLSCAASEFTFSNYAMSWVRQTPEKRLEWVATISSGG
SFTYYPDNLKGRFTVSRDNAKDTLYLQMSSLRSEDTAIYYCARQSLGYYFDSWGQ
GTTLTVSS

**F - Anti-CFH antibodies**

Complement Factor H (NM_000186) is a member of the Regulator of Complement Activation (RCA) gene cluster. The CFH protein contains twenty short consensus repeat (SCR) domains, is secreted into the bloodstream, and has an essential role in the regulation of complement activation, restricting this innate defense mechanism to microbial infections. Mutations in this gene have been associated with hemolytic-uremic syndrome (HUS) and chronic hypocomplementemic nephropathy. Complement factor H (CFH) is an inhibitor of the alternative complement pathway.

CFH detection, when combined with panels of the present invention, may further increase the reliability of the prediction.

Any anti-CFH antibody may be used to detect CFH. Specific examples of such antibodies include Bsi0077, Bsi0271, Bsi0885 and Bsi0893, as disclosed below.

Bsi0077

Bsi0077 is an IgG type monoclonal antibody. The heavy chain variable amino acid sequence is represented in SEQ ID NO: 83 reproduced below (CDRs underlined).

EVQLQQSGPVLVKPGASVKISCKTSGYTFTEYTIHWMRQSHGKSLEWIGGINPNKG
NTNFNQKFKGKATLTVDKSSSTAYMELHSLPSEDSAVFYCARANWDVYAVDSWG
QGTSVTVSS

Antigen binding was determined by sandwich reaction with BSI0271 and direct binding to purified natural CFH.

Bsi0271

Bsi0271 is an anti-CFH IgG type monoclonal antibody which binds a peptide sequence selected from SEQ ID NOs: 43-56.

Additional anti-CFH antibodies include IgG Bsi0893 and Bsi0885, which bind a peptide sequence presented below, respectively.

| Ab | | Sequence | SEQ ID |
|---|---|---|---|
| Bsi0893 | CFH | CLPYPLCRGTAT | 75 |
| | | TECLPYPLCLYN | 76 |
| | | NETWSEFWRLHN | 78 |
| | | TAQDDRIFHMMH | 79 |
| | | RGTQDMEYFAMH | 80 |

(continued)

| Ab | | Sequence | SEQ ID |
|---|---|---|---|
| Bsi0885 | CFH | TVNPLLRLLSMG | 64 |
| | | NNPMLVLLSHGA | 65 |
| | | TVPQPQRLTQKS | 66 |
| | | AKYESLLRMLAA | 67 |
| | | GHESGYHISERA | 68 |
| | | GHEEHLWQILHF | 69 |
| | | GHEEQRNWSMEG | 70 |
| | | STYTDMLADLSA | 71 |
| | | SYESLLMKLARG | 72 |
| | | YRDSPGPRDKLL | 73 |
| | | GHEEREYQMMLQ | 74 |

In a specific assay format, CFH can be detected using the following combination of reagents:

| Capture reagents | Detection Ab | Target Protein |
|---|---|---|
| Bsi0271 | Bsi0885 | CFH |
| Bsi0893 | Bsi0885 | CFH |

**G. Lung cancer biomarker combinations (Fig 1-3)**

We have measured the concentration of the six biomarkers in plasma of lung cancer patients and healthy subjects using sandwich ELISA assays. Hpt, ACT, and LRG-1 proteins were measured using SW ELISA assays (Fig. 1-3) and cytokeratin-19 was measured using a commercially available kit CYFRA 21-1 EIA (Fujirebio). The discrimination capabilities of the individual biomarkers are quite good and their potential as diagnostics tools was determined from the area (AUC) under the receiver operating characteristics curves (ROC). The two biomarkers with the best performance as determined by the AUC were LRG1 and CYFRA21-1 having an AUC of 0.85 and 0.91 respectively, followed by ACT (AUC of 0.81), C9 (AUC of 0.80), and Hpt (AUC of 0.74) (Table below). The sensitivity at 95% specificity was also quite different among the different biomarkers and ranged from 67% for CYFRA 21-1 and 62 % for LRG1 to 32 % for HPT. Combining CYFRA21-1 with each one of the other biomarkers showed increased diagnostic performance and therefore added value. Using support vector machines, and ten fold cross validation of the results generated with the samples from the clinical cohort III (training set), we have determined multiple marker classifiers composed of combinations between the five biomarkers. An increase of more than 10% in the sensitivity at 95% specificity of the prediction was achieved when CYFRA21-1 was added to either a panel of three biomarkers (LRG-1, C9, ACT) or four biomarker panel (ACT + C9 + LRG + Hpt). A remarkable performance was observed for a four-member panel with an AUC of 0.93 that could provide specificity of 95 % with a sensitivity above 84 %.In stages II and later, the panel reached 90 % sensitivity.

Table: Performance of combined biomarkers

| | Se at 95% Sp | AUC | | Se at 95% Sp | AUC |
|---|---|---|---|---|---|
| Cyfra | 67.39 | 0.91 | | | |
| LRG | 62.26 | 0.85 | LRG + Cyfra | 83.25 | 0.93 |
| C9 | 43.87 | 0.80 | C9 + Cyfra | 76.85 | 0.91 |
| ACT | 60.85 | 0.81 | ACT + Cyfra | 81.28 | 0.92 |
| Hpt | 32.08 | 0.74 | Hpt + Cyfra | 69.46 | 0.91 |
| ACT + C9 + LRG | 63.68 | 0.85 | ACT + C9 + LRG + Cyfra | 84.24 | 0.93 |
| ACT + C9 + LRG + Hpt + CFH | 72.17 | 0.90 | ACT + C9 + LRG + Hpt + CFH + Cyfra | 84.24 | 0.94 |

Se: sensitivity ; Sp : specificity

Binding peptides recognized by antibodies suitable for use in the disclosure.

| SEQ ID NO: | Peptide Sequence | Exemplary Antibody |
|---|---|---|
| 1 | **NNSYLDEEGTWL** | Bsi0358 |
| 2 | **FPTHNPTSPLDM** | |
| 3 | **DLNDPPFFTSVS** | |
| 4 | **TTHWDHPFYDNT** | |
| 5 | **AWHPSSPLDYGF** | |
| 6 | ANSVVYKPSSPL | |
| 7 | **TSYNTDHPFHYP** | |
| 8 | **SPSAPSSPLDSM** | |
| 9 | **VRACVDCDQPFY** | |
| 10 | **IAPSSFLDERTD** | |
| 11 | **TRPHTDPPFWWA** | |
| 12 | **EPDPPFYTHLTA** | |
| 13 | SQMKPSSHLDWD | |
| 14 | **HTLPQFLEWHKR** | Bsi0272 |
| 15 | **GQVVFQDWLLVR** | |
| 16 | **HENANKIAPWVQ** | |
| 17 | **QLKIAPWVIESP** | |
| 18 | **TDTLDSFLGRSP** | |
| 19 | **KPPRQWAPWVM** | |
| 20 | **IAQTLPAFLATR** | |
| 21 | **TETLHEFLDGRK** | |
| 22 | **KHLEHPTYHLWH** | |
| 23 | SLNTIAPWILTA | |
| 24 | **KATTHNIAWWVA** | |

(continued)

| SEQ ID NO: | Peptide Sequence | Exemplary Antibody |
|---|---|---|
| 25 | HDNINMAWWVEW | |
| 26 | EWYVSTSLLPLP | Bsi0352 |
| 27 | HIYYISESLLPM | |
| 28 | HVEYEIIEQMIY | |
| 29 | SPFFYTLTRVPM | |
| 30 | SSYLYTTQFVPL | |
| 31 | TGWMISTTLIPT | |
| 32 | YKYSYTTELILI | |
| 33 | SYTYTTHLLPLA | |
| 34 | TMTQYYYSTTHY | |
| 35 | QFWTITTTLVPH | |
| 36 | GPKMDALSKAEN | Bsi0033 |
| 37 | GPRWLDALFMAE | |
| 38 | HIGAHDSFETKH | |
| 39 | NNSYLDEEGTWL | Bsi0359 |
| 40 | AMNTVVCYDEPC | |
| 41 | TNSPLDELGTHP | |
| 42 | STNSHEHWPMSP | |
| 43 | NHFSSLGILTAA | Bsi0271 |
| 44 | IPQPSILTTPML | |
| 45 | LRLPNTYENAKN | |
| 46 | GPLSPAHLSQRN | |
| 47 | FPLPFDPEMLPL | |
| 48 | FPLPPEYSMKKD | |
| 49 | FKIPEGNNVSVL | |
| 50 | FRLPPHDSSDHH | |
| 51 | FFLPHSATLPSH | |
| 52 | FILPHRNGYIDT | |
| 53 | LKLPNPDTAAPT | |
| 54 | NNNMGNILLITP | |
| 55 | VLLVPHHSHQYD | |
| 56 | YLKESTHVLLAA | |
| 57 | HDDWYISTSHTD | Bsi0351 |
| 58 | SYTYTTHLLPLA | |
| 59 | EWYVYEKLFPLP | |
| 60 | YKYSYTTELILI | |
| 61 | HQYYYTSSLIYT | |
| 62 | TAWHTTEELIML | |

(continued)

| SEQ ID NO: | Peptide Sequence | Exemplary Antibody |
|---|---|---|
| 63 | NGYIFTTQLIWA | |

Peptides recognized by the antibodies used for the sandwich assays

| | | | |
|---|---|---|---|
| Bsi0186 | ACT | SVPWWTQSLLMS<br>DIWSTMEHNKYN<br>HINAIQYPLPHT | SEQ82<br>SEQ84<br>SEQ85 |

References

Stewart, B.W. & Kleihues, P. World cancer report. 1, 181-188 (2003).

Rivera, M.P., Detterbeck, F. & Mehta, A.C. Diagnosis of lung cancer: the guidelines. Chest 123, 129S-136S (2003).

Kulpa, J., Wojcik, E., Reinfuss, M. & Kolodziejski, L. Carcinoembryonic antigen, squamous cell carcinoma antigen, CYFRA 21-1, and neuron-specific enolase in squamous cell lung cancer patients. Clin Chem 48, 1931-1937 (2002).

Zolg, W. The proteomic search for diagnostic biomarkers: lost in translation? Mol Cell Proteomics 5, 1720-1726 (2006).

Beane, J., Spira, A. & Lenburg, M.E. Clinical impact of high-throughput gene expression studies in lung cancer. J Thorac Oncol 4, 109-118 (2009).

Rifai, N., Gillette, M.A. & Carr, S.A. Protein biomarker discovery and validation: the long and uncertain path to clinical utility. Nat Biotechnol 24, 971-983 (2006).

Gao, W.M. et al. Distinctive serum protein profiles involving abundant proteins in lung cancer patients based upon antibody microarray analysis. BMC Cancer 5, 110 (2005).

Knezevic, V. et al. Proteomic profiling of the cancer microenvironment by antibody arrays. Proteomics 1, 1271-1278 (2001).

Maneewatch, S. et al. Human single-chain antibodies that neutralize homologous and heterologous strains and clades of influenza A virus subtype H5N1. Antivir Ther 14, 221-230 (2009).

Houimel, M. & Dellagi, K. Isolation and characterization of human neutralizing antibodies to rabies virus derived from a recombinant immune antibody library. J Virol Methods (2009).

Uhlen, M. et al. A human protein atlas for normal and cancer tissues based on antibody proteomics. Mol Cell Proteomics 4, 1920-1932 (2005).

Blow, N. Antibodies: The generation game. Nature 447, 741-744 (2007).

Kohler, G. & Milstein, C. Continuous cultures of fused cells secreting antibody of predefined specificity. Nature 256, 495-497 (1975).

Shapiro, S.S.a.W., M. B. Biometrika 52, 591-611 (1965).

Chu PG et al., Histopathology 2002, 40:403-439.

Dohmoto K et al., Int J Cancer 2001, 91:468-473.

Stieber P., et al., Clin. Biochem., 26: 301-304, 1993, and Cancer (Phila.) 72: 707-713,1993.

Stieber et al., National. Academy of. Clinical Biochemistry Guidelines for the use of Tumor Markers in Lung Cancer, 1-29 2006.

Pujol J-L., et al., Cancer Res., 53: 61-66, 1993.

Ebert W et al., Eur. J. Clin. Chem. Clin. Biochem., 32: 189-199, 994.

Harlow et al., Antibodies: A laboratory Manual, CSH Press, 1988.

Kohler et al., Nature 256 (1975) 495.

Ward et al., Nature 341 (1989) 544.

## Claims

1. A method for the detection, diagnosis or staging of lung cancer in a patient, the method comprising the combined analysis of CYFRA 21-1 and LRG-1 proteins, in a blood sample from the subject, said combined analysis providing an indication of the presence, stage or progression of lung cancer in the patient:

2. The method of claim 1, wherein analysing said proteins comprises contacting the sample, or an aliquot thereof, with a first specific binding reagent that binds a CYFRA 21-1 protein and with at least a second specific reagent that binds a LRG-1 protein, and determining the presence or amount of protein bound to said binding reagents.

3. The method of claim 2, wherein said contacting with the at least 2 binding reagents is performed simultaneously or sequentially, on the same sample or on different aliquots of a same sample.

4. The method of claim 2 or 3, wherein the binding reagents are antibodies or fragments thereof retaining antigen specificity.

5. The method of claim 1, wherein lung cancer is Stage I or II lung cancer.

6. The method of claim 1, wherein lung cancer is Stage Ia or IIa lung cancer.

7. The method of claim 4, wherein the anti-LRG-1 antibody or a fragment thereof is an antibody or a fragment thereof which binds human LRG-1 and which binds a peptide having a sequence selected from SEQ ID NOs: 26-35 and 57-63.

8. The method of claim 4, wherein the anti-CYFRA 21-1 antibody or a fragment thereof is an antibody, or a fragment thereof which binds human cytokeratin 19 fragment, CYFRA21-1, or a protein or peptide comprising a sequence selected from SEQ ID NOs: 93-96.

9. The use of CYFRA 21-1 in combination with LRG-1 as protein biomarkers for the detection, diagnosis or staging of lung cancer in a blood sample from the subjects.

10. The use of claim 9, for the detection of Stage I LC, particularly Stage Ia,

11. The use of claim 9, for the detection of Stage II LC, particularly stage IIa.

## Patentansprüche

1. Verfahren zum Nachweis, Diagnose oder Einstufung von Lungenkrebs in einem Patienten, wobei das Verfahren die kombinierte Analyse von CYFRA 21-1 und LRG-1 Proteinen in einer Blutprobe des Subjektes umfasst, wobei die kombinierte Analyse einen Hinweis auf das Vorhandensein, dem Stadium oder dem Fortschritt von Lungenkrebs in dem Patienten liefert.

2. Das Verfahren nach Anspruch 1, wobei das Analysieren der Proteine das Kontaktieren der Probe oder eines Aliquots davon mit einem ersten spezifischen Bindungsreagenz, das ein CYFRA 21-1 Protein bindet und mit mindestens einem zweiten spezifischen Reagenz, das ein LRG-1 Protein bindet, umfasst, und Bestimmen einer Proteinmenge, die an die Bindungsreagenzien gebunden ist.

3. Das Verfahren nach Anspruch 2, wobei das Kontaktieren mit den mindestens zwei Bindungsreagenzien gleichzeitig oder der Reihe nach auf der gleichen Probe oder auf verschiedenen Aliquots der gleichen Probe durchgeführt wird.

4. Das Verfahren nach Anspruch 2 oder 3, wobei die Bindungsreagenzien Antikörper oder Fragmente davon, die die Antigenspezifität beibehalten haben, sind.

5. Das Verfahren nach Anspruch 1, wobei der Lungenkrebs ein Stadium I oder II Lungenkrebs ist.

6. Das Verfahren nach Anspruch 1, wobei wobei der Lungenkrebs ein Stadium Ia oder IIa Lungenkrebs ist.

7. Das Verfahren nach Anspruch 4, wobei der Anti-LRG-1-Antikörper oder ein Fragment davon ein Antikörper oder ein Fragment davon ist, der menschliches LRG-1 und ein Peptid mit einer Sequenz ausgewählt aus SEQ ID NO: 26-35 oder 57-63 bindet.

8. Das Verfahren nach Anspruch 4, wobei der Anti-CYFRA 21-1-Antikörper oder ein Fragment davon ein Antikörper oder ein Fragment davon ist, der menschliches Cytokeratin 19 Fragment, CYFRA 21-1, oder ein Protein oder Peptid umfassend eine Sequenz ausgewählt aus SEQ ID NO: 93-96 bindet.

9. Verwendung von CYFRA 21-1 in Kombination mit LRG-1 als Proteinbiomarker zum Nachweis, Diagnose oder Einstufung von Lungenkrebs in einer Blutprobe des Patienten.

10. Die Verwendung nach Anspruch 9, zum Nachweis von Stadium I LC, insbesondere Stadium Ia.

11. Die Verwendung nach Anspruch 9, zum Nachweis von Stadium II LC, insbesondere Stadium IIa.

## Revendications

1. Méthode pour la détection, le diagnostic ou l'évaluation du stade du cancer du poumon chez un patient, la méthode comprenant l'analyse combinée des protéines CYFRA 21-1 et LRG-1, dans un échantillon de sang provenant du sujet, ladite analyse combinée fournissant une indication de la présence, du stade ou de la progression du cancer du poumon chez le patient.

2. Méthode selon la revendication 1, dans laquelle l'analyse desdites protéines comprend la mise en contact de l'échantillon, ou d'une aliquote de celui-ci, avec un premier réactif de liaison spécifique qui se lie à une protéine CYFRA 21-1 et avec au moins un second réactif spécifique qui se lie à une protéine LRG-1, et la détermination de la présence ou de la quantité de protéine liée auxdits réactifs de liaison.

3. Méthode selon la revendication 2, dans laquelle ladite mise en contact avec les au moins deux réactifs de liaison est effectuée simultanément ou séquentiellement, sur le même échantillon ou sur différentes aliquotes d'un même échantillon.

4. Méthode selon la revendication 2 ou 3, dans laquelle les réactifs de liaison sont des anticorps ou des fragments de ceux-ci conservant la spécificité d'antigène.

5. Méthode selon la revendication 1, dans laquelle le cancer du poumon est un cancer du poumon au Stade I ou II.

6. Méthode selon la revendication 1, dans laquelle le cancer du poumon est un cancer du poumon au Stade Ia ou IIa.

7. Méthode selon la revendication 4, dans laquelle l'anticorps anti-LRG-1 ou un fragment de celui-ci est un anticorps ou un fragment de celui-ci qui se lie à LRG-1 humain et qui se lie à un peptide ayant une séquence choisie parmi SEQ ID NOs : 26-35 et 57-63.

8. Méthode selon la revendication 4, dans laquelle l'anticorps anti-CYFRA 21-1 ou un fragment de celui-ci est un anticorps, ou un fragment de celui-ci qui se lie au fragment de la cytokératine 19 humaine, CYFRA21-1, ou à une protéine ou un peptide comprenant une séquence choisie parmi SEQ ID NOs: 93-96.

9. Utilisation de CYFRA 21-1 en combinaison avec LRG-1 comme biomarqueurs protéiques pour la détection, le diagnostic ou l'évaluation du stade du cancer du poumon dans un échantillon de sang provenant du sujet.

10. Utilisation selon la revendication 9, pour la détection du cancer du poumon (LC) au Stade I, en particulier Stade Ia.

11. Utilisation selon la revendication 9, pour la détection du cancer du poumon (LC) au Stade II, en particulier Stade IIa.

Figure 1

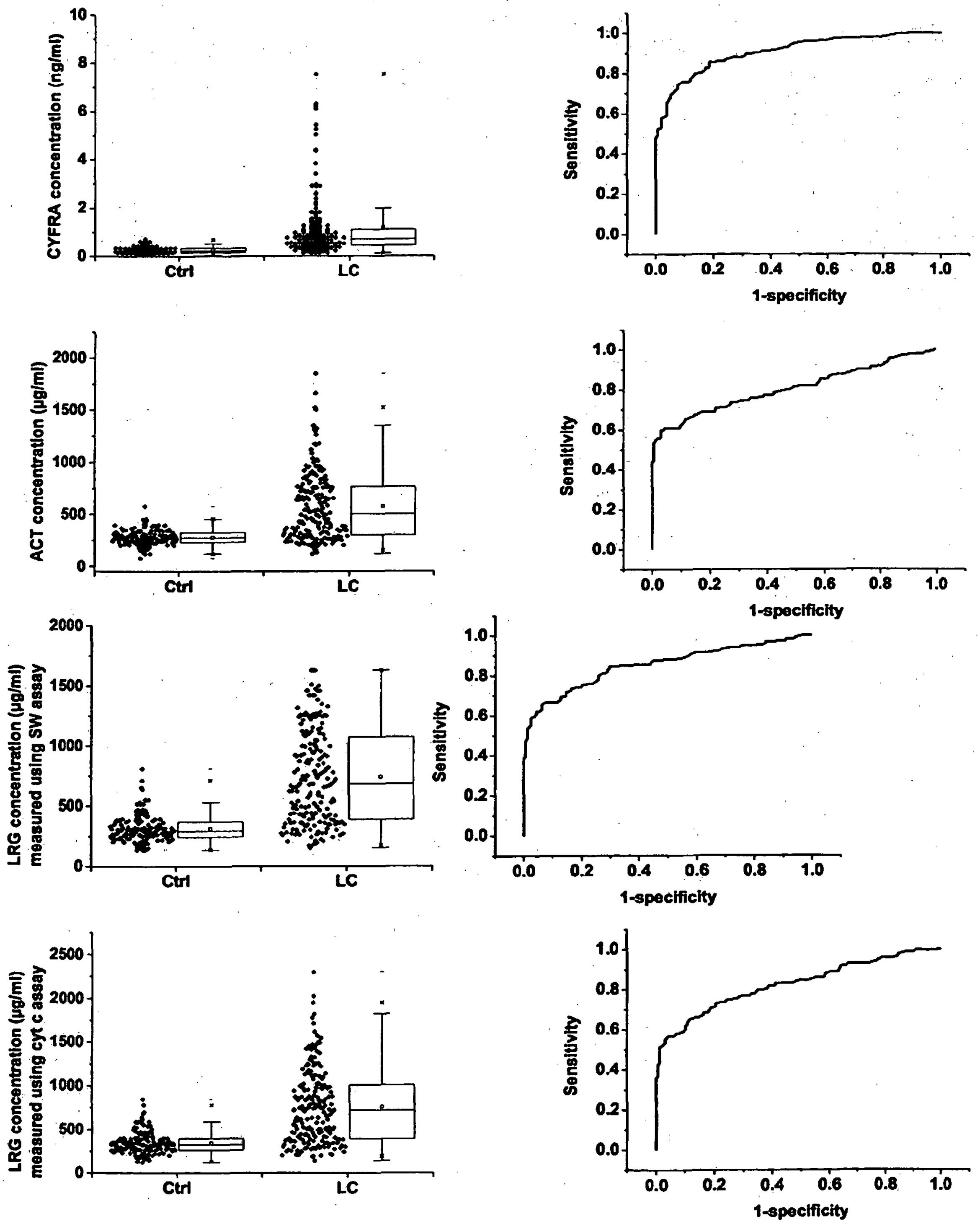
CYFRA concentration (ng/ml)
Ctrl
LC
Sensitivity
1-specificity
ACT concentration (µg/ml)
LRG concentration (µg/ml) measured using SW assay
LRG concentration (µg/ml) measured using cyt c assay

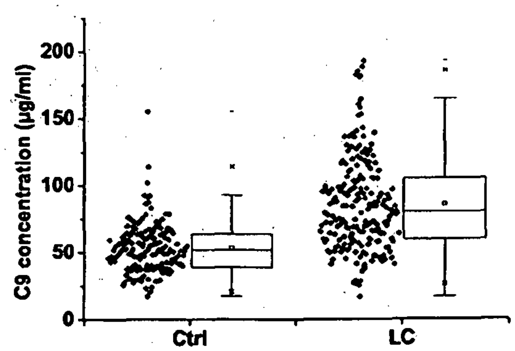
C9 concentration (µg/ml)
0
50
100
150
200
Ctrl
LC

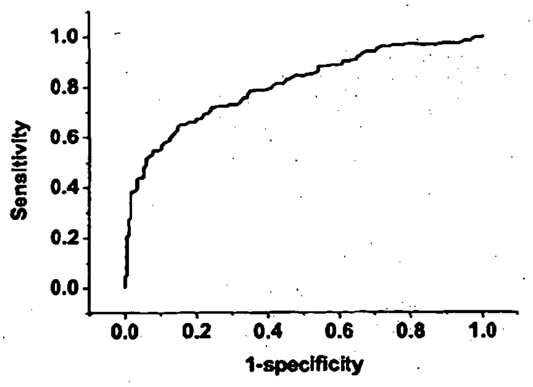
Sensitivity
0.0
0.2
0.4
0.6
0.8
1.0
1-specificity

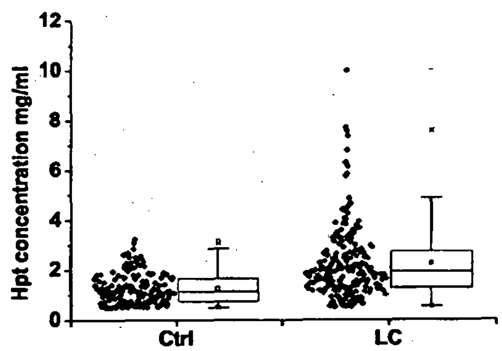
Hpt concentration mg/ml
0
2
4
6
8
10
12
Ctrl
LC

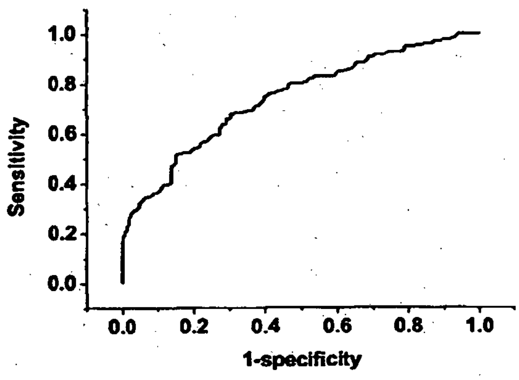
Sensitivity
0.0
0.2
0.4
0.6
0.8
1.0
1-specificity

Figure 2

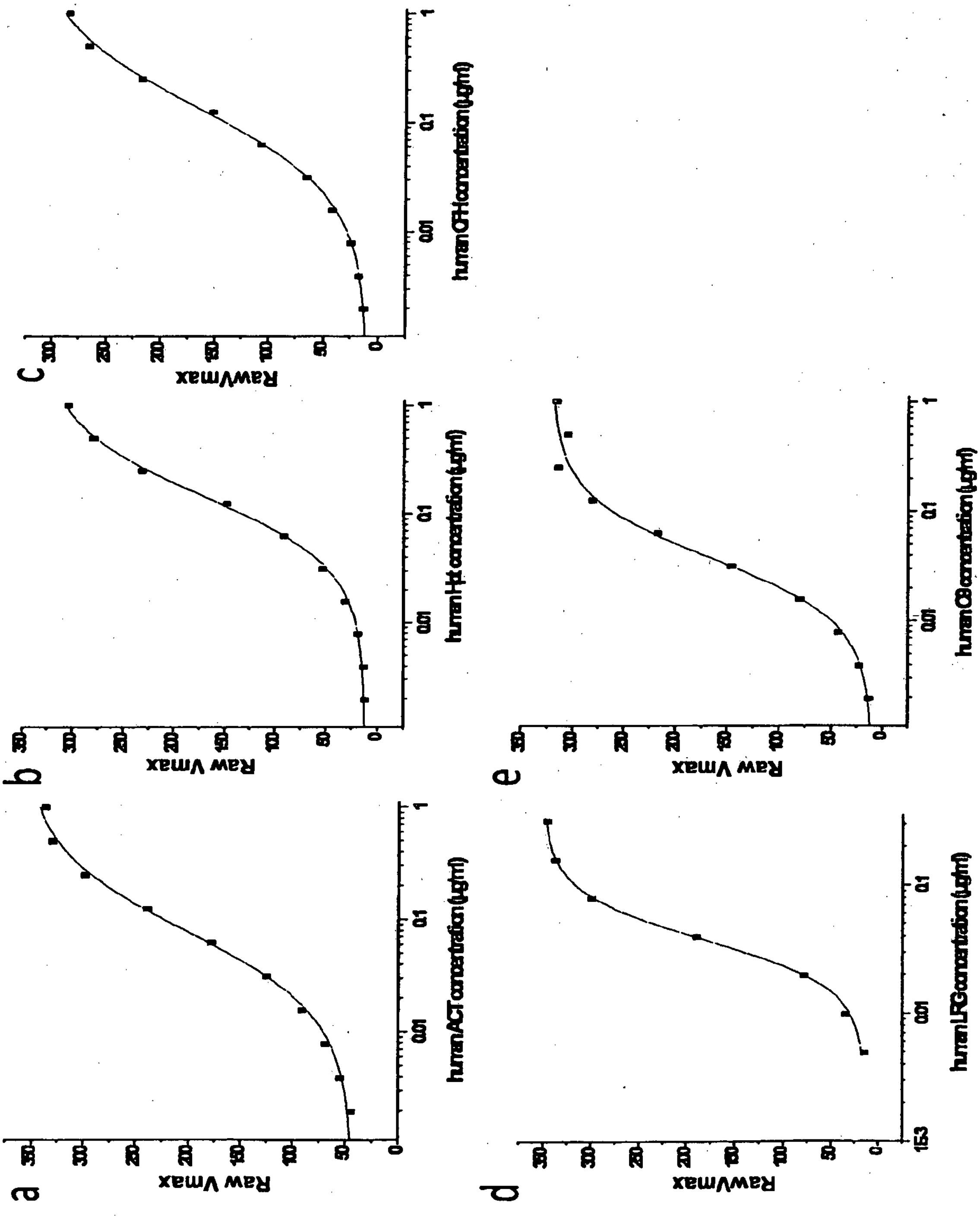
a
b
c
d
e
Raw Vmax
human ACT concentration (µg/ml)
human Hpt concentration (µg/ml)
human CFH concentration (µg/ml)
human LRG concentration (µg/ml)

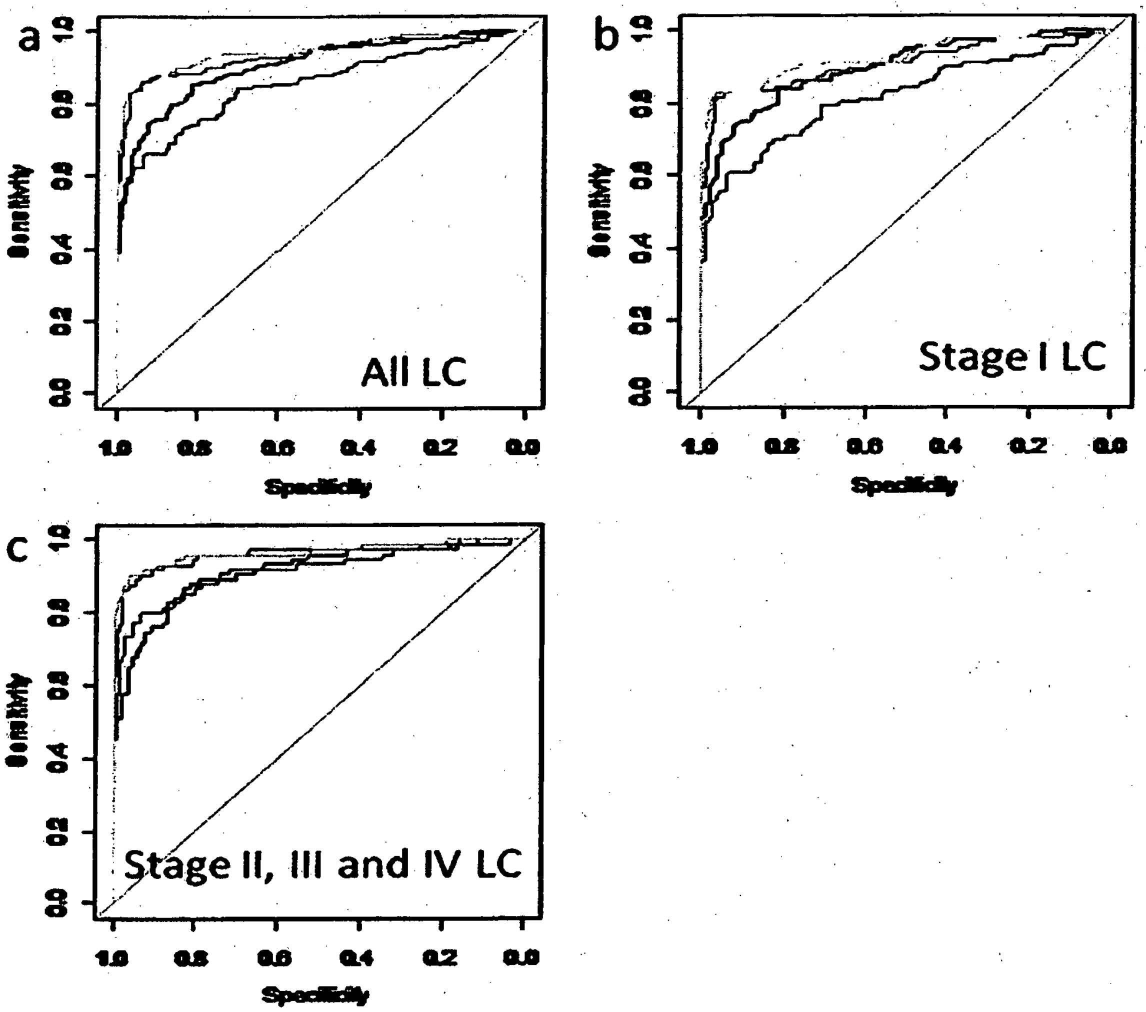
a
All LC
b
Stage I LC
c
Stage II, III and IV LC
Sensitivity
Specificity
1.0
0.8
0.6
0.4
0.2
0.0

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2010061354 W
- US 7416850 B


### Non-patent literature cited in the description


- **BÖTTGER V. et al.** *Eur J Biochem,* 1995, vol. 231 (2), 475-85
- **WEIVODA et al.** *J Immunol Methods,* 04 April 2008, vol. 336 (1), 22-9
- **WEIVODA S et al.** *J Immunol Methods,* 20 July 2008, vol. 336 (1), 22-9
- **STEWART, B.W. ; KLEIHUES, P.** *World cancer report.,* 2003, vol. 1, 181-188
- **RIVERA, M.P. ; DETTERBECK, F. ; MEHTA, A.C.** Diagnosis of lung cancer: the guidelines. *Chest,* 2003, vol. 123, 129S-136S
- **KULPA, J. ; WOJCIK, E. ; REINFUSS, M. ; KOLODZIEJSKI, L.** Carcinoembryonic antigen, squamous cell carcinoma antigen, CYFRA 21-1, and neuron-specific enolase in squamous cell lung cancer patients. *Clin Chem,* 2002, vol. 48, 1931-1937
- **ZOLG, W.** The proteomic search for diagnostic biomarkers: lost in translation?. *Mol Cell Proteomics,* 2006, vol. 5, 1720-1726
- **BEANE, J. ; SPIRA, A. ; LENBURG, M.E.** Clinical impact of high-throughput gene expression studies in lung cancer. *J Thorac Oncol,* 2009, vol. 4, 109-118
- **RIFAI, N. ; GILLETTE, M.A. ; CARR, S.A.** Protein biomarker discovery and validation: the long and uncertain path to clinical utility. *Nat Biotechnol,* 2006, vol. 24, 971-983
- **GAO, W.M. et al.** Distinctive serum protein profiles involving abundant proteins in lung cancer patients based upon antibody microarray analysis. *BMC Cancer,* 2005, vol. 5, 110
- **KNEZEVIC, V. et al.** Proteomic profiling of the cancer microenvironment by antibody arrays. *Proteomics,* 2001, vol. 1, 1271-1278
- **MANEEWATCH, S. et al.** Human single-chain antibodies that neutralize homologous and heterologous strains and clades of influenza A virus subtype H5N1. *Antivir Ther,* 2009, vol. 14, 221-230
- **HOUIMEL, M. ; DELLAGI, K.** Isolation and characterization of human neutralizing antibodies to rabies virus derived from a recombinant immune antibody library. *J Virol Methods,* 2009
- **UHLEN, M. et al.** A human protein atlas for normal and cancer tissues based on antibody proteomics. *Mol Cell Proteomics,* 2005, vol. 4, 1920-1932
- **BLOW, N.** Antibodies: The generation game. *Nature,* 2007, vol. 447, 741-744
- **KOHLER, G. ; MILSTEIN, C.** Continuous cultures of fused cells secreting antibody of predefined specificity. *Nature,* 1975, vol. 256, 495-497
- **SHAPIRO, S.S.A.W., M. B.** *Biometrika,* 1965, vol. 52, 591-611
- **CHU PG et al.** *Histopathology,* 2002, vol. 40, 403-439
- **DOHMOTO K et al.** *Int J Cancer,* 2001, vol. 91, 468-473
- **STIEBER P. et al.** *Clin. Biochem.,* 1993, vol. 26, 301-304
- *Cancer (Phila.),* 1993, vol. 72, 707-713
- **STIEBER et al.** *National. Academy of. Clinical Biochemistry Guidelines for the use of Tumor Markers in Lung Cancer,* 2006, 1-29
- **PUJOL J-L. et al.** *Cancer Res.,* 1993, vol. 53, 61-66
- **EBERT W et al.** *Eur. J. Clin. Chem. Clin. Biochem.,* 1994, vol. 32, 189-199
- **HARLOW et al.** Antibodies: A laboratory Manual. CSH Press, 1988
- **KOHLER et al.** *Nature,* 1975, vol. 256, 495
- **WARD et al.** *Nature,* 1989, vol. 341, 544